(19) Europäisches Patentamt
European Patent Office
Office européen des brevets

(11) Veröffentlichungsnummer: **0 169 466**
**B1**

# (12) EUROPÄISCHE PATENTSCHRIFT

(45) Veröffentlichungstag der Patentschrift: **04.04.90**

(51) Int. Cl.$^5$: **A 61 K 31/62**

(21) Anmeldenummer: **85108762.7**

(22) Anmeldetag: **12.07.85**

(60) **Teilanmeldung 89114991.6 eingereicht am 12/07/85.**

## (54) Kombinationspräparat aus Xanthinderivaten und O-Acetylsalicylsäure bzw. deren pharmakologisch verträglichen Salzen und dessen Verwendung.

(30) Priorität: **21.07.84 DE 3426961**
**07.03.85 DE 3508097**

(43) Veröffentlichungstag der Anmeldung:
**29.01.86 Patentblatt 86/05**

(45) Bekanntmachung des Hinweises auf die Patenterteilung:
**04.04.90 Patentblatt 90/14**

(84) Benannte Vertragsstaaten:
**AT BE CH DE FR GB IT LI LU NL SE**

(56) Entgegenhaltungen:
**EP-A-0 069 958**
**DE-A-2 831 728**
**FR-M- 7 217**
**GB-A-2 096 138**

(73) Patentinhaber: **HOECHST AKTIENGESELLSCHAFT**
**Postfach 80 03 20**
**D-6230 Frankfurt am Main 80 (DE)**

(72) Erfinder: **Weithmann, Klaus Ulrich, Dr.**
**Am Domherrenwald 18**
**D-6238 Hofheim am Taunus (DE)**
Erfinder: **Seiffge, Dirk, Dr.**
**Hauptstrasse 2**
**D-6309 Münzenberg (DE)**

**EP 0 169 466 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Das 1-(5-Oxohexyl)-3,7-dimethylxanthin (Pentoxifyllin) wird bekanntlich als pharmazeutisches Mittel zur Verbesserung der Fließfähigkeit des Blutes eingesetzt. Als Ursach hierfür wird angenommen (Deutsche Mediz. Wochenschrift *107* (1982), 1674), daß die Fließschubspannung des Blutes abnimmt, weil die Vorformbarkeit der Erythrozyten durch Pentoxifyllin-Therapie verbessert wird. Auch die Aggregation der Thrombozyten in vitro kann durch Pentoxifyllin inhibiert werde, allerdings erst in Konzentrationen, die über den zur medizinischen Therapie angewendeten liegen (IRCS (Med. Sci.) *8* (1980) 293, Thrombos. Haemostas. *46* (1981) 272).

Zudem ist beschreiben worden, daß mit Pentoxifyllin die Freisetzung einer antiaggregatorisch wirksamen Substanz aus Rattenaorten ex vivo durch Behandlung der Tiere mit Pentoxifyllin gesteigert werden kann. Ebenso ist bekannt, daß die Freisetzung der so stimulierten antiaggregatorisch wirksamen Substanz, mutmaßlich Prostacyclin, durch Behandlung der Ratten mit Acetylsalicylsäure unterdrückt werden kann (Vasa *10* (1981) 249). Weitergehende eigene Versuche haben dies bestätigt (siehe unten).

O-Acetylsalicylsäure ist als Hemmstoff der Aggregation menschlicher Blugplättchen bekannt (z.B. Br. J. clin. Pharmac. *7* (1979) 283), und es wurde berichtet, daß sie hinsichtlich der Vermeidung von Thrombosen und Schlaganfällen wertvolle therapeutisch Effekte zeigen kann (Blood *52* (1978) 1073, N. Engl. J. Med. *299* (1978). Als Wirkungsmechanismus ist beschreiben, daß Acetylsalicylsäure das in den Blutplättchen lokalisierte Enzym Cyclooxygenase hemmt (J. Clin. Invest. *56* (1975) 624) und somit die Biosynthese des die Aggregation fördernden Thromboxan $A_2$ inhibiert wird. Allerdings kann Acetylsalicylsäure auch die in der Gefäßwand befindliche Cyclooxygenase hemmen und damit die Synthese des die Aggregation hemmenden Prostazyklins. Da die Hemmung der vaskulären Cyclooxygenase aber erste bei höheren Dosen Acetylsalicylsäure zu beobachten ist (Pharmacol. Research Commun. *10* (1978) 759), wird konsequenterweise empfohlen, mit niedrigen Acetylsalicylsäure-Dosierungen eine antithrombotische Wirkung zu erreichen (Lancet, iii (1979) 1213, Prostaglandins and Medicine *4* (1980) 439). Es ist aber auch beschreiben worden, daß die antithrombotische Wirkung von Acetylsalicylsäure mit steigender Dosierung zunimmt und eine optimale Wirkung unter Bedingungen erreicht wird, bei denen sowohl die Prostacyclinals auch die Thromboxan-Biosynthese weitgehend gehemmt wird (Prostaglandins, Leukotrienes and Medicine *12* (1983) 235).

Es ist auch schon bekannt, die günstige Wirkung von Acetylsalicylsäure einerseits und die des Xanthinderivates 7-(2-Diäthylaminoäthyl)-theophyllin andererseits zu addieren, indem man das Säure-Base-Addukt aus den beiden Einzelsubstanzen herstellt und medizinisch anwendet (GB—OS—2 096 138). Auch die Beeinflussung der Überlebenszeit von Thrombocyten in Patienten mit künstlichen Herzklappen durch kombinierte gleichzeitige Anwendung von Pentoxifyllin und Acetylsalicylsäure ist bekannt (Singapore Med. Journal *20* Suppl. (1979) 30).

Es wurde nun gefunden, daß eine mit zeitlichem Abstand aufeinanderfolgende Anwendung von A) Xanthinderivaten bzw. deren wirksamen Metaboliten einerseits und B) Acetylsalicylsäure bzw. deren pharmakologisch verträglichen Salzen andererseits in einer bestimmten Reihenfolge eine überaus starke Verbesserung der Therapie von Krankheiten ermöglicht, die durch gestörte Blutbestandteile, insbesondere Thrombocyten bzw. Erythrocyten, aber auch Leukocyten verursacht bzw. gekennzeichnet sind. Die zeitlich abgestufte Anwendung der Xanthinderivate, insbesondere Pentoxifyllin, die erste nach 10 Minuten bis 4 Stunden von der Anwendung der Acetylsalicylsäure bzw. ihres Salzes gefolgt wird, führt zu viel stärkeren Effekten als wenn die Kombination aus den beiden Einzelsubstanzen gleichzeitig verabfolgft wird, wo sogar eine Verminderung dieser Wirkungf eintritt. Dies ist umso überasschender, als die gleichzeitige Verabreichung von Xanthinderivaten, wie Pentoxifyllin, und Acetylsalicylsäure nur zu solchen antithrombotischen und antiaggregatorischen Wirkungen führt, wie man sie bei der Einzelgabe der Acetylsalicylsäure erhalten hätte (vgl. unten Tabellen 1 und 2).

Gegenstand der Erfindung sind daher Kombinationspräparate, enthaltend A) Xanthinderivate der Formeln (I) bzw. (II), (s. Patentanspruch 1) bze. Pro-Drugs von Oxoalkyl- bzw. Hydroxyalkylxanthinen, bzw. deren wirksame Metabolite und B) O-Acetylsalicylsäure bzw. deren pharmakologisch verträgliche Salze mit oder ohne C) einen pharmazeutischen Träger zur zeitlich abgestuften Anwendung in der Therapie von Krankheiten, die durch gestörte Blutbestandteile, insbesondere Thrombocyten bzw. Erythrocyten, aber auch Leukocyten verursacht bzw. gekennzeichnet sind, derart, daß die Komponente A) zuerst freigesetzt wird. Mit anderen Worten eignen sich die erfindungsgemäßen Mittel aufgrund ihrer überadditiven Effekte zur antithrombotischen, blutflußfördernden, entzündungshemmenden, schmerzstillenden, antiaggregatorischen und cytostatischen Therapie bzw. Prophylaxe. Gegenstand der Erfindung ist daher auch die Verwendung von A) Xanthinderivaten der Formel I bzw. II bzw. von Prodrug-Formen der Oxoalkylxanthine der Formeln I und II bzw. der Hydroxyalkylxanthine der Formel I, bzw. deren Metaboliten und B) O-Acetylsalicylsäure bzw. deren pharmakologisch verträglichen Salzen, C) mit oder ohne pharmazeutische Träger zur Herstellung von Mitteln, die eine zeitlich abgestufte Freisetzung in der Weise bewirken, daß die Komponente A) zuerst freigesetzt wird, gegen Krankheiten, die durch gestörte Blutbestandteile verursacht sind, Gegenstand der Erfindung ist weiter die Herstellung von pharmazeutischen Zubereitungen gemäß Anspruch 3 und die Verwendung des Mittels in der Human- bzw. Veterinärmedizin. Durch die erfindungsgemäßen Kombinationspräparate wird es möglich, daß das Xanthinderivat bereits vor der Acetylsalicylsäure freigesetzt wird, d.h. bioverfügbar ist.

Es ist ein besonderer Vorteil, daß wegen des überadditiven Effektes bei der aufeinanderfolgenden Anwendung die anzuwendenden Mengen an Xanthinderivat und Acetylsalicylsäure auf solche Mengen reduziert werden können, die bei ihrer alleinigen Verabreichung eine nur minimale pharmakologische Wirkung zeigen, so daß gleichzeitig Nebenwirkungen, die von hohen Dosen dieser Arzneimittel hervorgerufen werden, verringert werden. Dies ist deswegen von großer Bedeutung, weil bekanntlich Acetylsalicylsäure in den üblichen Dosierungen unerwünschte Nebenwirkungen (z.B. British Journal of Clinical Pharmacology 1980, *10,* Suppl. 2 und International Meeting on Side Effects of Antiinflammatory, Analgesic Drugs, Verona, 13.—15.9.82, Abstracts) wir Asthma, allergische Urtikaria, analgetische Nephropathie sowie Magen- und Darmulcera hervorrufen kann. Auch die Xanthinderivate können unerwünschte Nebenwirkungen zeigen. Durch das erfindungsgemäße Kombinationspräparat kann nun überraschenderweise die erforderliche Dosierung an Acetylsalicylsäure beim Menschen drastisch reduziert werden und auch die Menge an Xanthinderivat, so daß die allgemeine toxikologische Verträglichkeit noch weiter verbessert wird. (s. unten).

Geeignete Xanthinderivate sind z.B. 1,3,7-trisubstituierte Verbindungen der Formel I, in der einer der Reste $R^1$ und $R^3$ eine geradkettige Alkyl-, (ω-1)-Oxoalkyl- oder (ω-1)-Hydroxyalkylgruppe mit 3 bis 8 C-Atomen und die beiden anderen Reste $R^2$ und $R^3$ oder $R^1$ und $R^2$ geradkettige oder verzweigte Alkylgruppen mit 1 bis 8 C-Atomen in der Position von $R^1$ und $R^3$ und 1 bis 4 C-Atomen in der Position von $R^2$ darstellen, wobei die Summe der C-Atome dieser beiden Alkylsubstituenten höchstens 10 beträgt.

Bevorzugt sind dabei solche Xanthinverbindungen der Formel I, in denen $R^1$ oder $R^3$ einen Alkyl-, (ω-1)-Oxoalkyl- oder (ω-1)-Hydroxyalkylrest mit 5 oder 6 C-Atomen bedeutet und die beiden Alkylsubstituenten $R^2$ und $R^3$ bzw. $R^1$ und $R^2$ zusammen 2 bis 6 C-Atome umfassen.

Unter diesen Verbindungen sind weiderum jene besonders bevorzugt, die in der position von $R^1$ oder $R^3$ eine Hexyl-, 5-Oxohexyl- oder 5-Hydroxyhexylgruppe tragen. Zu ihnen gehören insbesondere 1-Hexyl-3,7-dimethylxanthin, 1-(5-Hydroxyhexyl)-3,7-dimethylxanthin, 1-(5-Oxohexyl)-3,7-dimethyl-xanthin, 1,3-Dimethyl-7-(5-hydroxyhexyl)-xanthin, 1,3-Dimethyl-7-(5-oxohexyl)-xanthin, 1-(5-Hydroxyhexyl)-3-methyl-7-propylxanthin und 1-(5-Oxohexyl)-3-methyl-7-propylxanthin.

Eine andere geeignete Xanthingruppe sind die Verbindungen der Formel II, in der R für einen Alkylrest mit 1 bis 4 C-Atomen steht.

Die Oxoalkylxanthine der Formeln I und II und die Hydroxyalkylxanthine der Formel I brauchen nicht per se eingesetzt zu werden, sondern können auch in Prodrug-Form eingesetzt werden, aus der erste im Organismus durch Biotransformation die therapeutisch wirksamen Xanthinverbindungen mit den in Formeln I und II definierten Substituenten freigesetzt werden können. Hierfür kommen beispeilsweise die acetalisierten Oxoalkylxanthine in Frage, in denen die Carbonylgruppe durch das Strukturelement der Formel (III) (seihe Patentanspruch 6), ersetzt ist, und die O-acylierten Hydroxyalkylxanthine mit dem Strukturelement der Formel $R^6$—CO—O— (IV) anstelle der Hydroxyfunktion, wobei $R^4$ und $R^5$ jeweils eine Alkylgruppe mit bis zu 4 C-Atomen oder zusammen eine Äthylen-, Trimethylen- oder Tetramethylengruppe darstellen und $R^6$ einen Alkylrest mit bis zu 4 C-Atomen, Phenyl, substituiertes Phenyl, Pyridyl oder substituiertes Pyridyl bedeuten.

Geeignete pharmakologisch verträgliche Salze der Acetylsalicylsäure sind solche mit pharmakologisch verträglichen Metallkationen, Ammonium, Aminkationen oder quaternären Ammoniumkationen. Bevorzugt sind solche der Alkalimetalle, wie Lithium, Natrium und Kalium, und der Erdalkalimetalle, wie Magnesium und Calcium, obgleich auch kationische Formen anderer Metalle, wie Aluminium, Zink und Eisen verwendet werden können.

Pharmakologisch verträgliche Aminkationen sind solche von primären, sekundären oder tertiären Aminen wie der Alkylamine, z.B. Methyl-, Dimethyl-, Trimethyl-, Äthyl-, Dibutyl-, Triisopropyl-, N-Methylhexyl, Benzyl-, β-Phenyläthylamin, Äthylendiamin, Diäthylentriamin, Piperidin, Morpholin, Piperazin, Mono-, Di- und Triäthanolamin, Äthyldiäthanolamin, N-Butyläthanolamin und dergleichen. Geeignete Aminsalze sind auch die basischen Aminsalze des Lysins und des Arginins. Geeignete pharmakologisch verträgliche quaternäre Ammoniumkationen sind z.B. das Tetramethylammonium, Tetraäthylammonium und das Benzyltrimethylammonium.

Die Xanthinderivate einerseits und die Acetylsalicylsäure-Komponente andererseits können zur Erzielung der überadditiven Wirkung auch gleichzeitig verabreicht werden, wobei aber eine Verabreichung in Dosiseinheiten in getrennter Form bevorzugt ist, wenngleich die Komponenten auch in Gemischen in einer geeigneten Form, die eine zeitlich aufeinanderfolgende Anwendung ermöglichen, verabreicht werden können. Die Dosiseinheiten können in Form von festen Arzneiformen, wie Kapseln (einschließlich Mikrokapseln, die im allgemeinen keine pharmazeitischen Träger enthalten), Tabletten (einschließlich Dragees und Pillen), oder Zäpfchen vorliegen, wobei bei Verwendung von Kapseln das Kapselmaterial die Funktion des Trägers wahrnimmt und der Inhalt z.B. als Pulver, Gel, Emulsion, Dispersion, Lösung vorliegen kann. Besonders vorteilhaft und einfach ist es jedoch, orale und perorale Formulierungen mit den beiden Wirkstoffen herzustellen, die die berechneten Mengen der Wirkstoffe zusammen mit jedem gewünschten pharmazeutischen Träger enthalten und die so beschaffen sind, daß die Freisetzung der Wirkstoffe zeitlich abgestuft (sequentiell) erfolgt. Auch eine entsprechende Formulierung (Zäpfchen) für die rektale Therapie kann angewandt werden. Ebenso ist die transdermale und parenterale (intraperitoneale, intravenöse, subkutane, intramuskuläre) Injektion von Lösungen, z.B. mittels geeigneter Mehrkammer-Injektionseinheiten möglich.

Derartige Kombinationspräparate können nach üblichen Verfahren hergestellt werden. Die erfindungsgemäße sequentielle Freigabe (Bioverfügbarkeit) der Wirkstoffe kann erreicht werden, indem man in üblicher Weise, z.B. nach Sucker, Fuchs und Speiser, Pharmazeutische Technologie, Stuttgart 1978, S. 424, Tabletten, Pillen oder Granulatkörper, die als Arzneimittel Acetylsalicylsäure oder deren medizinisch verträgliche Salze, wie D,L-Lysin-monoacetylsalicylat enthalten, mit einem Mantel überzeiht, der als Wirkstoff z.B. Pentoxifyllin enthält, vorteilhaft in Kombination mit Schleimbildnern, Harzen wie Polystyrol, oder anderen üblichen, die Verträglichkeit fördernden Mitteln. die als Kern verwendeten Tabletten, Pillen oder Granulatkörper können nach üblichen Verfahren hergestellt werden und enthalten Trägermittel und andere übliche Hilfsstoffe, wie Stärke, z.B. Kartoffel-, Mais- oder Weizenstärke, Zellulose bzw. deren Derivate, insbesondere mikrokristalline Zellulose, Siliziumdioxid, verschiedene Zucker wie Milchzucker, Magnesiumcarbonat und/oder Kalziumphosphate. Der z.B. Pentoxifyllin enthaltende Mantel kann z.B. mit Hilfe der in der pharmazeutischen Technologie üblichen Verfahren, wie Preß-, Tauch- oder Wirbelbettverfahren, oder durch Kesseldragierung auf den Kern aufgezogen werden. Die Dragierlösung besteht gewöhnlich aus Zucker- und/oder Stärkesirup, unter Zusatz von Gelatine, Gummi arabicum, Polyvinylpyrrolidon, synthetischen Zelluloseestern, oberflächenaktiven Substanzen, Weichmachern, Pigmenten und ähnlichen Zusätzen entsprechend dem Stand der Technik.

Die sequentielle Freigabe der Wirkstoffe kann auch mit Hilfe von Schichttabletten (worunter auch Punkttabletten verstenden werden), ebenfalls beschreiben in Sucker et al., l.c. erreicht werden, in denen die schneller zu resorbierende Schicht das Xanthin enthält, vorteilhaft ebenfalls in Kombination mit Schleimbildnern oder anderen üblichen, die Verträglichkeit fördernden Mitteln. Bei dieser Arzneiform kann die Freisetzung der Wirkstoff durch unterschiedliche Freigaberaten aus den Tablettenschichten durch Verwendung geeigneter üblicher Hilfsstoffe, wie sie z.B. oben aufgeführt sind, erreicht werden. Eine abgestufte Freigabe der beiden Wirkstoffe, z.B. Pentoxifyllin und Acetylsalicylsäure, kann auch erreicht werden, indem die die Acetylsalicylsäure enthaltende Komponente Retardierungsmittel, gegebenenfalls auch in Form von permeablen Membranen, wie solche auf Zellulose- oder Polystyrolharzbasis, oder Ionenaustauscher enthält oder in Form, von Mikrokapseln eingesetzt wird, die magensaftresistent sind oder eine verzögerte Freisetzung ermöglichen. Es kann aber auch der Acetylsalicylsäure enthaltende Kern mit einem Überzug, beispielsweise bestehend aus Polymethacrylsäureestern (Eudragit®) versehen werden, der eine verzögerte Freigabe ermöglicht.

Zur Herstellung der Arzneiformen kann jedes der üblichen Fließregulierungs-, Schmier- bzw. Gleitmittel wie Magnesiumstearat und Trennmittel verwednet werden.

Das Gewichtsverhältnis der Acetylsalicylsäure zu den Xanthinderivaten, wie Pentoxifyllin, kann innerhalb weiter Grenzen variieren. Das genaue, auf eine bestimmte Kombination anzuwendende Verhältnis kann leicht mit Hilfe der weiter unten beschreibenen experimentellen Verfahren ermittelt werden. Im allgemeinen beträgt der Gewichtsanteil der Xanthinderivate, beispielsweise Pentoxifyllin, bezogen auf ein Gewichtsteil Acetylsalicylsäure, mindestens zwischen etwa 1,0, vorzugsweise mindestens etwa 0,3—0,5 und insbesondere mindestens etwa 2 und höchstens etwa 50, vorzugsweise höchstens etwa 10. Auch der optimale Zeitabstand zwischen der Anwendung des Xanthinderivates und der Acetylsalicylsäure bzw. die optimale Freisetzungsrate aus den galenischen Zubereitungen können durch Anwendung dieser experimentellen Verfahren ermittelt werden. Zunächst wird das Xanthinderivat, beispielsweise Pentoxifyllin, freigesetzt und 15 Minuten bis 4 Stunden später die Acetylsalicylsäurekomponente. Besonders bevorzugt ist ein Zeitabstand von etwa 20 bis 90 Minuten und insbesondere zwischen 30 und 60 Minuten. Die anzuwendende Dosierung ist selbstverständlich abhängig von verschiedenen Faktoren, wie dem zu behandelnden Lebewesen (d.h. Menschen oder Tieres, Alter, Gewicht, allgemeiner Gesundheitszustand), der Schwere der Symptoms, der zu be hendelnden Erkrankung, (falls vorhanden) der Art der begleitenden Behandlung mit anderen Arzneimitteln, der Häufigkeit der Behandlung usw. Die Dosierungen werden im allgemeinen bis zu fünfmal pro Tag und vorzugsweise einmal bis dreimal pro Tag verabreicht. Das Gewichtsverhältnis der Einzelwirkstoff soll innerhalb des oben angegebenen Bereiches liegen und die Menge der Bestandteile innerhalb des für das zu behandelnde Lebwesen verträglichen, wirksamen Dosierungsbereichs.

Z.B. beträgt die bevorzugte Dosis Acetylsalicylsäure beim Menschen bei alleiniger Verabreichung zwei- bis dreimal täglich 500 bis 2000, insbesondere 1000 mg. Die bevorzugte Dosis Pentoxifyllin beträgt beim Menschen bei alleiniger Verabreichung zwei- bis dreimal täglich 200 bis 800, insbesondere 300 mg bis 600 mg. Aus diesen Gewichtsverhältnissen für das Verhältnis Acetylsalicylsäure/Pentoxifyllin können die jeweiligen Mengen genau berechnet werden. Eine geeignete Therapie besteht somit z.B. in der Verabreichung von einer, zwei oder mehr, vorzugsweise 3 bis 8 Einzeldosierungen der erfindungsgemäßen Kombinationspräparate von je 100 bis 600, vorzugesweise mindestens 200 und insbesondere bis 400 mg Xanthinderivat, insbesondere Pentoxifyllin und 10 bis 2000 z.B. bis 400 mg Acetylsalicylsäure bzw. die entsprechende Menge eines Salzes, wobei die Menge naturgemäß von der Zahl der Einzeldosierungen und auch der zu behandelnden Krankheit abhängig ist und eine Einzeldosierung z.B. auch aus mehreren, gleichzeitig verabreichten Tabletten bestehen kann. Jedoch ermöglicht es die Erfindung auch in den Fällen, in denen die Verabreichung von besonders geringen Acetylsalicylsäuremengen (z.B. 10—50 mg je Tag oder weniger) gewünscht wird, gute Ergebnisse zu erhalten, und zwar wesentlich bessere als bei alleiniger Anwendung der gleichen Menge Acetylsalicylsäure.

Die erfindungsgemäßen Mittel können in gleicher Weise wie bekannte antithrombotische und die Blutplättchenaggregation inhibierende Mittel angewandt werden. In vivo-Anwendungen umfassen die Verabreichung an Menschen und Tieren, um die Bildung von arteriellen und venösen Blutgerinnseln zu verhindern, wie beispielsweise zur Verhinderung von vorübergehenden ischämischen Anfällen und bei der Langzeitprophylaxe nach Herzinfarkten und Schlaganfällen, sowie bei Arterioskerlose, aber auch bei der Operationsnachbehandlung zur Verhinderung postoperativer Thrombosen sowie bei der Nachbehandlung von Krebs zur Verhinderung bzw. Verminderung der Metastasenwirkung. Auch die Anwendung bei Patienten, die an Herz-Lungen-Maschinen sowie an die Nierendialyse angeschlossen sind, ist möglich, ebenso bei Patienten mit künstlichen Herzklappen, Gefäßprothesen, usw. Natürlich ist auch die Anwendung für eigentlichen Indikationen der Einzelbestandteile, z.B. Durchblutungsförderung (Claudicatio intermittens) sowie schmerzstillende und entzündungshemmende Wirkung (auch bei chronischen Entzündungen) möglich. In entzüdungshemmenden Präparaten liegt das Verhältnis von Xanthinderivat zur Komponente B im allgemeinen zwischen 0,1 und 1 und für andere Präparate im allgemeinen zwichen 0,5 und 50.

In vivo Untersuchungen:

Es wurde die Kombination von Pentoxifyllin und Acetylsalicylsäure in vivo mit Hilfe einer Versuchsanordnung bewertet, bei der eine intravaskuläre Thrombose mit einem Laser in den Arteriolen des Mesenteriums einer Ratte erzeugt wurde. Diese Arbeitsweise ist ein geeignetes experimentelles Modell für das erfindungsgemäße Kombinationspräparat. Die Auswertung erfolgte mittels vitalmikroskopischer Analyse (Nature, *218* (1968) 887 und Haemostasis *13* (1983) 61 und IRCS Med. Sci. *12* (1984) 91).

Die Testsubstanzen wurden in 0,9% Natriumchlorid-Lösung (enthielt 1% Carboxymethylzellulose (Serva, Heidelberg) appliziert, und zwar entweder per os, intraperitoneal oder intravenös. Kontrolltiere werden in entsprechender Weise, aber ohne Testsubstanzen behandelt. Als Versuchsteiere wurden männliche oder weibliche Sprague Dawley- oder Wistar-Ratten verwendet.

Die Untersuchung mit Pentoxifyllin, anderen Xanthinderivaten und Acetylsalicylsäure im Laser-induzierten Thrombosemodell erfolgte an weiblichen Sprague-Dawley-Ratten mit einem Körpergewicht von ca. 200 g. Die zu untersuchenden Tiere wurden mit 0,1 mg Atropinum sulf. sol. s.c. prämediziert und mit 100 mg Ketaminhydrochloride und 4 mg Xylazin pro kg KG i.p. anaesthetisiert. Zur Untersuchung dienten Arteriolen und Venolen des mit entgastem Paraffinöl überschichteten Mesenteriums mit einem Durchmesser von ca. 13 µm. Der Strahl des 4 W Argon-Lasers (Fa. Spectra Physics, Darmstadt,) wurde mittels einer Strahladaptions- und -justieranlage (Fa. BTG, München,) koaxial in den invertierten Strahlengang eines Mikroskopes (ICM 405, LD-Epipland 40/0.60; Fa. Zeiss, Oberkochen,) eingebracht. Die benutzte Wellenlänge beträgt 514,5 nm mit einer Energie oberhalb des Objektives von 30.5 mW. die Expositionszeit pro Einzelschuß dauert 1/15 sec. Alle Meßvorgänge wurden per Videokamera (Trinicon-Röhre, Sony, Köln) aufgenommen und auf einem Rekorder (Sony, U-matic $\frac{3}{4}$") gespeichert. Die Testsubstanzen wurden den Versuchstieren in verschiedenen Dosierungen oral eine Stunde, bei i.v. Gabe 10 min vor Versuchsbeginn verabreicht, Kontrolltiere erhielten die gleiche Menge des Placebos. Die Applikation der Substanzen erfolgte: 1) als Einzelgabe 2) zusammen als Kombination oder 3) zuerst Acetylsalicylsäure und nach 1 h Pentoxifyllin oder ein anderes Xanthinderivat sowie 4) zuerst Pentoxifyllin oder ein anderes Xanthinderivat und nach 1 h Acetylsalicylsäure (Tab. 1a). Tab. 1b zeigt den Einfluß verschiedener Zeitintervalle. Tab. 1c faßt die Wirkungen anderer Xanthinderivate zusammen.

Auswertung:

Es wird die Zahl der Schüsse gezählt, um einen definierten Thrombus zu induzieren. Die Schußfrequenz beträgt eine Läsion alle 2 min, wobei alle während des Beobachtungszeitraumes gebildeten Thromben von einer Mindestgröße von $\frac{1}{4}$ Gefäßradius ausgezählt und vermesen wurden.

Die statistische Auswertung der Versuchsergebnisse erfolgte mit Hilfe des $\chi^2$-Testes (L. Cavalli-Sforza, Biometrie, Stuttgart, 1969, S. 49 ff).

Ergebnisse:

Die Ergebnisse sind in Tabellen 1a-c dokumentiert. Die Einzelgaben von 5 mg/kg per os von Acetylsalicylsäure oder Pentoxifyllin sind nicht signifikant wirksam, wobei aber Pentoxifyllin schon eine 20%-ige Hemmung der Thrombusbildung zeigt. In der Dosis von 10 mg/kp per os besitzen beide Substanzen eine signifikante Wirkung. Die gleichzeitige Verabreichung von Pentoxifyllin und Acetylsalicylsäure ergab keine Wirkung im Lasermodell. Dies war auch der Fall, wenn zuerst Acetylsalicylsäure und nach 1 h Pentoxifyllin verabreicht wurde. Hingegen besitzt die Anwendung von zuerst Pentoxifyllin und nach 1 h Acetylsalicylsäure eine dosisbezogene, signifikante Wirkung im Laser-induzierten Thrombosemodell an Arteriolen und Venolen des Rattenmesenteriums. Der über-additive Effekt dieser zeitlich abgestufften Anwendung im Vergleich zur Einzelgabe dokumentiert sich deutlich in der prozentualen Veränderung im Hinblick auf die Kontrolle (Tab. 1a).

Die in Tab. 1b aufgelisteten Ergebnisse zeigen, daß die Zeitspanne zwischen den beiden Einzelgaben breit gewählt werden kann, das Optimum liegt zwischen 15 und 180 Minuten. Auch mit anderen Xanthinderivaten (s. Patentansprüche) können die erfindungsgemäßen Effekte erzielt werden, wenn sie zeitlich versetzt mit Acetylsalicylsäure verabreicht werden. Antithrombotische Effekte einer Auswahl solcher Xanthinderivate sind in Tab. 1c demonstriert.

Die zeitlich abgestufte Anwendung kann mit Hilfe einer handelsüblichen Perfusoreinheit mit zwei getrennt steuerbaren Kammern (z.B. der Fa. Braun, Melsungen; mit über Zeitschltuhr getrennt gestaltetem Motorvorschub) durchgeführt werden. Die beiden Kammern der Perfusoreinheit wurden jeweils mit Pentoxifyllin-Lösung (entsprechend 10 mg Pentoxifyllin/kg Ratte) bzw. mit Acetylsalicylsäure-Lösung (entsprechend 1 mg/kg) gefüllt (Lösungsmittel seihe oben). 20 min nach der Injektion der Pentoxifyllin-Lösung in die Schwanzvene erfolgte die Schaltuhr-gesteuerte Injektion der Acetylsalicylsäure-Lösung. Im Vergleichsexperiment wurden die beiden Kammern gleichzeitig injiziert. Die Ergebnisse entsprachen den nach oraler Gabe erhaltenen Meßwerten, d.h. durch die zeitlich abgestufte Anwendung wurden Wirkungen erhalten, die weit über den bei gleichzeitiger Verabreichung erhaltenen liegen.

Ex vivo Untersuchungen:

Die Thrombozytenaggregation wurde nach an sich bekannten Verfahren bestimmt. Männliche Kaninchen (eigene Zucht, BASK, SPF Wiga ca. 2,5 bis 3,5 kg) wurden intravenös (Ohrvene) mit Pentoxifyllin und/oder DL-Lysinmonoacetylsalicylat, gelöst in physiologischer Kochsalzlösung, behandelt. Hierauf wurde Blut aus der Ohrvene gezogen, mit einer 3,5% igen Trinatriumzitratlösung im Verhältnis 9:1 versetzt und 45 Minuten bei Zimmertemperatur inkubiert. Sodann wurde 10 Minuten bei 1000 Umdrehungen pro Minute zentrifugiert. Die obere Schicht, die das plättchenreiche Plasma bildet, wurde abgetrennt und die untere Schicht 10 Minuten bei 28 000 Umdrehungen pro Minute zentrifugiert. Nun enthielt die obere Schicht das plättchenarme Plasma, das ebenfalls abgetrennt wurde. Das plättchenreiche Plasma wurde mit Hilfe des plättchenarmen Plasma auf etwa 6 bis $7 \cdot 10^8$ Plättchen/ml verdünnt (Coulter Counter, Coulter Electronics, Krefeld). Die Plättchenaggregation wurde optisch durch Messung der Lichttransmission in einem Born'schen Aggregometer (Fa. Labor GmbH, Hamburg) verfolgt. Das Volumen des Testansatzes war 0,25 ml, die Temperatur 37°C. die Aggregationsinduktion erfolgte mit $2 \cdot 10^{-4}$M Arachidonsäure (Serva, Heidelberg), gereinigt unter Schutzgas (Argon) über präparative Hochdruckflüssigkeitschromatographie (HPLC Reversed Phase C-18-Säule). Die Zunahme der Thrombozytenaggregation wurde an Hand der Lichttransmission verfolgt. Meßgröße in diesem System ist die maximale Aggregationsamplitude E. Die Ergebnisse sind in Tabelle 2 niedergelegt.

Weibliche Ratten (Hoe Wiskf, ca. 180 g) wurden durch orale Applikation mit folgenden Medikamenten (in Polyäthylenglykol (PEG), MG 400 entsprechend 1 ml/kg) behandelt:

Experiment 1.) 30 mg/kg Pentoxifyllin, nach 30 Min. 3 mg/kg Acetylsalicylsäure.
Experiment 2.) 30 mg/kg Pentoxifyllin, nach 30 Min. 10 mg/kg Acetylsalicylsäure.
Experiment 3.) 3 mg/kg Acetylsalicylsäure, nach 30 Min. 30 mg/kg Pentoxifyllin.
Experiment 4.) 10 mg/kg Acetylsalicylsäure, nach 30 Min. 30 mg/kg Pentoxifyllin.
Experiment 5.) 30 mg/kg Pentoxifyllin, nach 30 Min. nur Solvens.
Kontrollexperiment 6.) 1 ml/kg PEG 400 ohne Medikamente.

Nach 18 Stunden wurden diese Prozeduren wiederholt und die Ratten sodann 1 Stunde nach der letzten Applikation unter Äthernarkose getötet und die thorakale Aorta entnommen. Aortasegmente wurden sofort bei 24°C in 3 ml gepufferter 0,09 M NaCl-Lösung, pH=7,5, 30 Min. lang inkubiert. Aliquote der Aorta-Überstände wurden sodann als Hemmstoffe der mit Adenosindiphosphat induzierten Aggregation von Humanthrombozyten wie folgt eingesetzt: Gesund erscheinenden männlichen und weiblichen Freiwilligen, die im vorangegangenen Zeitraum von 10 Tagen keine Medikamente eingenommen hatten, wurde durch vorsichtige Kanülierung der Antekubitalvene Blut entnommen, das sofort mit Natriumzitrat (ad 0,38%) stabilisiert wurde. Durch 15-minütiges Zentrifugieren bei 140 × g erhielt man im Überstand plättchenreiches Plasma (PRP), dessen Thrombozytengehalt im Bereich 2,5—$3{,}5 \cdot 10^8$/ml (Coulter Counter) lag. die Plättchenaggregation wurde optisch durch Messung der Lichttransmission in einem Born'schen Aggregometer (Fa. Labor GmbH, Hamburg) verfolgt. Das Gesamtvolumen des Teatansatzes war 0,25 ml. Das Plasma wurde mit den Aorta-Überständen 5 Min. bei 37°C vorinkubiert, die Aggregationsinduktion erfolgte sodann mit $2 \cdot 10^{-6}$M Adenosindiphosphat. Aus den jeweiligen maximalen Aggregationsamplituden wurden Dosis-Wirkungskurven in Abhängigkeit vom Aortagewicht erstellt und daraus graphisch die antiaggregatorische Aktivität im Überstand von 0,1 mg Aorta ermittelt. Die Aortagewichte wurden durch Wägen der 20 Stunden bei 60°C getrockneten Aorten bestimmt. Die Aggregationsmessungen erfolgten im Zeitraum von 1—2 Stunden nach Blutentnahme.

Toxizitätsprüfung. Methode:

Ratten wurden oral, wie oben beschrieben, jedoch mit steigender Dosierung, behandelt. Eine Gruppe erhielt Pentoxifyllin, eine zweite Gruppe erhielt Pentoxifyllin und zusätzlich Acetylsalicylsäure im Gewichtsverhältnis 10:1. Die letale Dosis wurde nach üblichen Standardverfahren (Litchfield u. Wilcoxon, 1949) als $LD_{50}$ berechnet:

$LD_{50}$ (Pentoxifyllin) = 1400 mg/kg
$LD_{50}$ (Pentoxifyllin/acetylsalicylsäure) = 1400 mg/kg.

Ergebnis:

Die toxikologische Verträglichkeit war für beide Gruppen gleich. Das bedeutet, daß das Verhältnis von pharmazeutischer Dosierung zu letaler Dosierung bei der erfindungsgemäßen Kombination Pentoxifyllin/

Acetylsalicylsäure viel kleiner und damit wesentlich günstiger ist, als bei Gabe von Pentoxifyllin oder Acetylsalicylsäure alleine.

Prüfung auf Magenverträglichkeit. Methode:

Auf Hunger gesetzte männliche Sprague-Dawley Ratten von 200—300 g wurden oral wie oben beschrieben mit Pentoxifyllin und nach einer Stunde mit Acetylsalicylsäure oder nur mit Acetylsalicylsäure behandelt. 24 Stunden nach der letzten Medikation wurde der Magen entlang der kleinen Kurvatur aufgeschnitten, unter fließendem Wasser gereinigt und auf Schleimhautläsionen inspiziert. Als Ulcera gelten alle makroskopisch sichtbaren Läsionen der Mucosa im Drüsenmagen.

Ergebnis:

Die gastrale Ulcerogenität von Acetylsalicylsäure wird durch Vorbehandlung mit Pentoxifyllin nicht beeinflußt (Tab. 4). Dies bedeutet, daß das Verhältnis von pharmazeutischer Dosierung zu magenunverträglicher Dosierung bei der erfindungsgemäßen Kombination (Pentoxifyllin-Gabe vor Acetylsalicylsäure) wesentlich kleiner und günstiger ist als bei Gabe von Acetylsalicylsäure alleine, da gemäß Tabelle 1 zur Erzielung gleicher antithrombotischer Effekte wesentlich größere Mengen an Acetylsalicylsäure notwendig sind.

Untersuchungen im Modell der chronischen Entzündung

Die erfindungsgemäßen Kombinationen wurden im pathologischen Rattenmodell der Adjuvans-Arthritis (induziert mit Mycobacterium butyricum) gemäß Clinical Hemorheology *3* (1983) 469—480 hinsichtlich ihrer blutrheologischen, antithrombotischen, antiaggregatorischen und antiinflammatorischen Wirkung nach 21 tägiger oraler Gabe untersucht und mit den entsprechenden Einzelsubstanzen vergleichen. Die Blutentnahme aus der thorakalen Aorta erfolgte 1 Stunde nach der letzten Substanzapplikation. Die blutrheologische Wirkung wurde in allen Einzelheiten bestimmt wie in Clinical Hemorheology *4* (1984) 263—273 beschreiben. Es wurde die Erythrozytenverformbarkeit im Filtrometer (MF 4, Fa. Myrenne, Roetgen, Deutschland) durch Auswertung der initialen Steigerung der Fließkurve quantifiziert. Tab. 5a zeigt, daß die, verglichen mit gesunden Kontrollratten, in Arthritisratten herabgesetzte Erythrozytenfiltrierbarkeit durch Acetylsalicylsäure und Pentoxifyllin wieder gesteigert werden kann. Schon die gleichzeitige Kombination der beiden Substanzen, insbesondere aber die konsekutive Anwendung (Pentoxifyllin 1 Stunde vor Acetylsalicylsäure) zeigt überadditive Effekte.

Die antithrombotische Wirkung wurde im Laser-Modell gemessen, wie oben beschreiben. Tab. 5b zeigt die Ergebnisse. Während in den gesunden Kontrolltieren durchschnittlich 2,173 (=100%) Laserschüsse zur Erzielung eines Thrombus gesetzt werden müssen, reichen im Arthritis-Tier 0,99 (=46%) Schüsse aus, d.h. im kranken Tier ist die Thromboseneigung erhöht. Tab. 5b zeigt, daß die Thromboseneigung durch die Behandlung mit den Medikamenten herabgesetzt wird und insbesondere durch Anwendung der erfindungsgemäßen Kombination den im gesunden tier gefundenen Werten angeglichen wird.

Die Messungen zur Thrombozytenaggregation erfolgten wie oben ausführlich beschrieben. Statt Arachidonsäure wurde jedoch 0,04 mg Collagen zur Induktion der Thrombozytenaggregation in 1 ml plättchenreichem Plasma (Aggregometer PA II, Fa. Myrenne, Roetgen) verwendet. Die Aggregationsamplitude (Aggregationsneigung) bei der unbehandelten arthritischen Ratte ist am höchsten (=100%), bei der gesunden Ratte tritt mit 0,04 mg Collagen keine Aggregation auf (Tab. 5c). Die aufgelisteten Ergebnisse zeigen, daß die pathologisch gesteigerte Aggregationsneigung in der arthritischen Ratte durch die genannten Medikamente vermindert werden kann. Die konsekutive Anwendung (Pentoxifyllin, 1 Stunde später Acetylsalicylsäure) ziegt wieder überadditive Effekte.

Die antiinflammatorische Wirkung wurde, wie oben zitiert, an Hand des Volumens des Pfotenoedems und des Standard-Nekroseindex quantifiziert. Unter Behandlung mit den Medikamenten nimmt die Nekrose- und Oedembildung deutlich ab. Tab. 6 zeigt die relative Besserung der Symptome in den behandelten Tieren, verglichen mit den unbehandelten Arthritis-Ratten. Die erfindungsgemäße konsekutive Anwendung (Pentoxifyllin, 1 Stunde später Acetylsalicylsäure) zeigt wieder überadditive Effekte.

Pharmazeutische Zubereitungen

Statt oder oben beschriebenen i.v.-Injektion mittels Perfusoreinheit konnten zur Erzielung überadditiver Effekte auch Suspensionen und feste Zubereitungen, die für die orale, perorale und rektale Applikation geeignet sind, verwendet werden.

Beispiele solcher Zubereitungen für die Anwendung am Menschen enthalten x mg Pentoxifyllin oder andere Xanthinderivate (siehe Beispiele 1—17) als Reinsubstanz und/oder als handelsübliche Fertigzubereitung (Trental®, Fa. Albert Roussel Pharma GmbH, Wiesbaden oder auch Rentylin®, Fa. Dr. Rentschler Arzneimittel GmbH & Co., Laupheim (abgekürzt T bzw. R)), oder Teile dieser Fertigzubereitungen, kombiniert mit y mg Acetylsalicylsäure, die auch an basische Ionenaustauscher (Dowex® 1 × 8, und QAE-Sephadex® (Serva, Heidelberg)) oder an Adsorborharz (Amberlite® XAD 2) gebunden sein können oder in Form von im Handel erhältlichen Mikrokapseln (Colfarit®, Bayer AG, Leverkusen (abgekürzt C)) oder Kristallen (R 95 D und M 80 D der Fa. Röhm Pharma GmbH, Weiterstadt, Deutschland) vorliegen. Die pharmazeutischen Träger dieser Kombinationen sind durch Erhitzen verfestigte Gele: (a) 20

Gewichtsprozent Gelatine/ 1 Gewichtsprozent Glycerin in Wasser sowie b) 1 Gewichtsprozent Agarose in Wasser sowie c) 10 Gewichtsprozent Äthylcellulose T50 (Hercules GmbH, Hamburg) in Aceton/Wasser (80:20 Gew.%), jeweils mit und ohne 8 Gewichtsprozent eingerührtes Pentoxifyllin oder anderes Xanthinderivat oder aber handelsübliche Gelatinekapseln (für die Anwendung am Menschen und Großtieren Größe 0 (Fa. Kapsugel, Basel)).

Die pharmazeutischen Zubereitungen (siehe Beispiele 1—17) werden in 10 ml Magensaft des Hundes bzw. 10 ml 0,1 N HCl eingegeben und unter schonendem Rühren in vitro bei 37°C gehalten. Aliquote des Überstandes werden zu gewissen Zeitintervallen entnommen und mittels Hochdruckflüssigkeitschromatographie (Säule: Rad pak C18 (Waters GmbH, Eschborn, Deutschland) 100 × 8 mm, 10 µm, Laufmittel: 300 ml Methanol/1 ml Essigsäure, 700 ml Wasser, Fließgeschwindigkeit: 1,5 ml/min.) aufgetrennt und die Bestandteile mit UV-Detektion bei 280 nm quantifiziert. In analoger Weise werden die pharmazeutischen Zubereitungen in Duodenalflüssigkeit (Hund) bzw. Natriumhydrogencarbonatlösung (pH = 7,4) eingesetzt.

Für die Anwendung am Kleintier (s. Tab. 1, Ratte) werden die Bestandteile der in den Beispielen 1—17 genannten Zubereitungen jeweils auf 1/200 der Gewichte reduziert bzw. Kapseln der Größe 4 und 5 verwendet.

| Beispiel Nr. | Pharmazeutische Träger | Acetylsalicylsäure-Gehalt | Xanthinderivat-Gehalt (total) | % Acetylsalicylsäure freigesetzt nach (. . .min) | % Xanthinderivat bei pH = 1,8 |
|---|---|---|---|---|---|
| 1 | Kapsel | 58 mg (M80D) | 350 mg (7-(2-Oxopropyl)-1,3-di-n-butyl-xanthin | 0 (15)<br>0 (30)<br>2 (90) x) | 77 (15)<br>99 (30) |
| 2 | Kapsel | 33 mg (M80D) | 100 mg 1-Hexyl-3,7-dimethyl-xanthin | 0 (15)<br>0 (30) x) | 79 (15)<br>98 (30) |
| 3 | Kapsel | 45 mg (M80D) | 400 mg Pentoxifyllin | 0 (15)<br>0 (30) x) | 79 (15)<br>99 (30) |
| 4 | Mantel: Gelatine, enthaltend 205 mg Xanthin<br>Kern: C | 500 mg (C) | 205 mg 1-(5-Hydroxyhexyl)-3,7-dimethyl-xanthin | 0 (5)<br>0 (10)<br>2,6 (15) | 2,5 (5)<br>15 (10)<br>28 (15) |
| 5 | Mantel: Gelatine, enthaltend 195 mg hexyl)Xanthin<br>Kern: R95D | 105 mg (R95D) | 195 mg 1-(5-Hydroxyhexyl-3,7-dimethyl-xanthin | 0 (5)<br>0 (10)<br>4 (15)<br>22 (60) | 6 (5)<br>21 (10)<br>35 (15)<br>76 (60) |
| 6 | 1.Schicht: 1 g Gelatine, enthaltend M80D<br>2. Schicht: R | 408 mg (M80D) | 400 mg Pentoxifyllin | 0 (15)<br>0 (30)<br>0 (60) x) | 10 (15)<br>20 (30)<br>40 (60) |
| 7 | 1. Schicht: Agarose, enthaltend, 70 mg Xanthin plus M80D<br>2. Schicht: R | 85 mg (M80D) | 470 mg Pentoxifyllin | 0 (60) x) | 30 (60) |

| Beispiel Nr. | Pharmazeutische Träger | Acetylsalicylsäure-Gehalt | Xanthinderivat-Gehalt (total) | % Acetylsalicylsäure freigesetzt nach (. . .min) | % Xanthinderivat bei pH = 1,8 |
|---|---|---|---|---|---|
| 8 | Mantel: Agarose, enthaltend 190 mg Xanthin<br>Kern: Acetylsalicylsäure, Dowex-gebunden | 62 mg (Dowex 1×8) | 190 mg 1-(5-Hydroxyhexyl)-3-methyl-7-propyl-xanthin | 0 (5)<br>0 (10)<br>3 (15) | 4 (5)<br>18 (10)<br>31 (15) |
| 9 | Mantel: Gelatine, enthaltend 280 mg Xanthin<br>Kern: Acetylsalicylsäure | 30 mg | 280 mg 1-(5-Oxohexyl)-3-methyl-7-propyl-xanthin | 0 (5)<br>5 (10)<br>30 (15) | 3 (5)<br>18 (10)<br>35 (15) |
| 10 | Suspension aus 1 ml Xanthinlsg. (50 mg/ml) und M80D | 530 mg (M80D) | 50 mg 1,3-Dimethyl-7-(5-hydroxyhexyl)-xanthin | 0 (5)<br>0 (15)<br>2 (20) | 4 (5)<br>20 (15)<br>35 (20) |
| 11 | Mantel: Gelatine, enthaltend 100 mg Xanthin<br>Kern: Acetylsalicylsäure, Amberlite gebunden | 95 mg (Amberlite) | 100 mg 1,3-Dimethyl-7-(5-oxohexyl)-xanthin | 0 (5)<br>0 (15)<br>2 (20) | 4 (5)<br>20 (15)<br>35 (20) |
| 12 | Kapsel | 390 mg (M80D) | 180 mg Pentoxifyllin | 0 (15)<br>0 (30)<br>0 (60)<br>0 (90) x) | 10 (15)<br>20 (30)<br>38 (60)<br>46 (90) |

| Beispiel Nr. | Pharmazeutische Träger | Acetylsalicylsäure-Gehalt | Xanthinderivat-Gehalt (total) | % Acetylsalicylsäure freigesetzt nach (. . .min) | % Xanthinderivat bei pH = 1,8 |
|---|---|---|---|---|---|
| 13 | Mantel: Gelatine enthaltend 65 mg Pentoxifyllin und 100 mg M80D<br>Kern: Pentoxifyllin (T) | 108 mg (M80D) | 465 mg Pentoxifyllin | 0 (60) x) | 18 (60) |
| 14 | Kapsel | 35 mg (M80D) | 200 mg Pentoxifyllin (T)<br>60 mg Pentoxifyllin | 0 (30)<br>0 (60) x) | 28 (30)<br>39 (60) |
| 15 | Kepsel | 62 mg (M80D) | 200 mg Pentoxifyllin (T) | 0 (30)<br>0 (60)<br>0 (75) x) | 19 (30)<br>29 (60)<br>36 (75) |
| 16 | 1. Schicht: Pentoxifyllin (R)<br>2. Schicht: 1 g Agarose, enthaltend M80D | 82 mg (M80D) | 400 mg Pentoxifyllin | 0 (30)<br>0 (60) x) | 20 (30)<br>38 (60) |
| 17 | 1. Schicht: Pentoxifyllin (R)<br>2. Schicht: Gelatine, enthaltend 190 mg Pentoxifyllin und M80D | 11 mg (M80D | 590 mg Pentoxifyllin | 0 (30)<br>0 (60) x) | 32 (30)<br>39 (60) |

x) Die magensäureunlöslichen Acetylsalicylsäure-Kristalle M80D werden bei pH = 7,4 quantitativ gelöst.

TABELLE 1a

Wirkung von Pentoxifyllin und/oder Acetylsalicylsäure bei verschiedener Applikationsfolge auf die Laser-induzierte Thrombose

| Substanz | Dosis mg/kg Rattengewicht p.o. | Anzahl Tiere n | Anzahl der Läsionen/ Tier | Anzahl der Schüsse $\bar{x}$ | Anzahl der Schüsse SEM | Veränderungen gegen Kontrolle absolut | Veränderungen gegen Kontrolle % | $\chi^2$-Test |
|---|---|---|---|---|---|---|---|---|
| Kontrolle | — | 12 | 48 | 2,17 | 0,01 | — | — | — |
| Acetysalicylsäure | 1 | 6 | 24 | 1,88 | 0,35 | −0,35 | −13 | |
| Acetylsalicylsäure | 5 | 6 | 24 | 1,79 | 0,20 | −0,38 | −18 | |
| Acetylsalicylsäure | 10 | 6 | 24 | 2,92 | 0,20 | 0,75 | 35 | p<0,01 |
| Pentoxifyllin | 5 | 6 | 24 | 2,63 | 0,24 | 0,46 | 21 | |
| Pentoxifyllin | 10 | 6 | 24 | 3,33 | 0,36 | 1,16 | 54 | p<0,01 |
| Pentoxifyllin+ Acetylsalicylsäure | 5 +5 | 6 | 24 | 2,42 | 0,26 | 0,25 | 12 | |
| Pentoxifyllin+ Acetylsalicylsäure | 10 +1 | 6 | 23 | 2,52 | 0,26 | 0,35 | 16 | |
| Pentoxifyllin+ Acetylsalicylsäure | 10 +10 | 6 | 24 | 2,25 | 0,26 | 0,08 | 4 | |
| Acetylsalicylsäure nach 1 h Pentoxifyllin | 5 +5 | 6 | 24 | 1,96 | 0,19 | −0,21 | −10 | |
| Acetylsalicylsäure nach 1 h Pentoxifyllin | 1 +10 | 7 | 28 | 2,36 | 0,23 | 0,19 | 9 | |
| Acetylsalicylsäure nach 1 h Pentoxifyllin | 10 +10 | 6 | 24 | 2,17 | 0,21 | 0 | 0 | |

TABELLE 1a (Fortsetzung)

Wirkung von Pentoxifyllin und/oder Acetylsalicylsäure bei verschiedener Applikationsfolge auf die Laser-induzierte Thrombose

| Substanz | Dosis mg/kg p.o. | Anzahl Tiere n | Anzahl der Läsionen/ Tier | Anzahl der Schüsse $\bar{x}$ | Anzahl der Schüsse SEM | Veränderungen gegen Kontrolle absolut | Veränderungen gegen Kontrolle % | $\chi^2$-Test |
|---|---|---|---|---|---|---|---|---|
| Pentoxifyllin nach 1 h Acetylsalicylsäure | 5 +5 | 6 | 24 | 3,21 | 0,21 | 1,04 | 48 | p<0,01 |
| Pentoxifyllin nach 1 h Acetylsalicylsäure | 10 +1 | 6 | 24 | 3,88 | 0,33 | 1,71 | 79 | p<0,01 |
| Pentoxifyllin nach 1 h Acetylsalicylsäure | 10 +10 | 6 | 24 | 4,71 | 0,35 | 2,54 | 117 | p<0,01 |
| Pentoxifyllin | 30 | 6 | 24 | 3,97 | 0,34 | 1,8 | 83 | |
| Acetylsalicylsäure | 30 | 6 | 24 | 3,21 | 0,2 | 1,04 | 48 | |
| Pentoxifyllin nach 1 h Acetylsalicylsäure | 30 +1 | 6 | 24 | 4,86 | 0,34 | 2,69 | 124 | p<0,01 |
| Pentoxifyllin nach 1 h Acetylsalicylsäure | 30 +10 | 6 | 24 | 4,83 | 0,37 | 2,66 | 123 | p<0,01 |
| Acetylsalicylsäure (Sephadex®) | 2 | 6 | 24 | 2,50 | 0,27 | 0,33 | 15 | |
| Pentoxifyllin + Acetylsalicylsäure (Sephadex®) | 10 +2 | 6 | 24 | 3,95 | 0,32 | 1,78 | 82 | p<0,01 |

TABELLE 1a (Fortsetzung)

Wirkung von Pentoxifyllin und/oder Acetylsalicylsäure bei verschiedener Applikationsfolge auf die Laser-induzierte Thrombose

| Substanz | Dosis mg/kg Rattengewicht p.o. | Anzahl Tiere n | Anzahl der Läsionen/ Tier | Anzahl der Schüsse $\bar{x}$ | Anzahl der Schüsse SEM | Veränderungen gegen Kontrolle absolut | Veränderungen gegen Kontrolle % | $\chi^2$-Test |
|---|---|---|---|---|---|---|---|---|
| Zubereitung analog Beispiel 3 Säureunlösliche mikroverkapselte Acetylsalicylsäure (M80D) | 10 | 6 | 24 | 2,79 | 0,19 | 0,62 | 29 | |
| Pentoxifyllin + säureunlösliche mikroverkapselte Acetylsalicylsäure (M80D) | 10<br>+10 | 6 | 24 | 4,28 | 0,32 | 2,11 | 97 | p<0,01 |
| Zubereitung analog Beispiel 6 säureunlösliche mikroverkapselte Acetylsalicylsäure (M80D) | 10 | 6 | 24 | 2,78 | 0,20 | 0,63 | 29 | |
| Pentoxifyllin + säureunlösliche mikroverkapselte Acetylsalicylsäure (M80D) | 10<br>10<br>30 | 6 | 24 | 4,88 | 0,35 | 2,71 | 125 | p<0,01 |

TABELLE 1b

Wirkung von Pentoxifyllin und Acetylsalicylsäure auf die Laser-induzierte Thrombose

Einfluß des Zeitintervalls zwischen der Verabreichung der Substanzen

Dosierung jeweils 10 mg/kg Pentoxifyllin p.o., dann nach der angegebenen Zeit 1 mg/kg Acetylsalicylsäure p.o.

| Substanz | Anzahl der Tiere | Anzahl der Läsionen | $\bar{x}$ | SEM | % Veränderung gegen Kontrolle | $\chi^2$-Test |
|---|---|---|---|---|---|---|
| Kontrolle (Placebo) | 8 | 32 | 2,19 | 0,02 | — | — |
| Pentoxifyllin, ohne Acetylsalicylsäure | 9 | 36 | 3,27 | 0,32 | +49 | p<0,05 |
| Acetylsalicylsäure, ohne Pentoxifyllin | 9 | 36 | 1,90 | 0,34 | −13 | |
| 0 min | 9 | 36 | 2,18 | 0,13 | 0 | |
| 15 min | 5 | 20 | 3,42 | 0,21 | +56 | p<0,05 |
| 30 min | 5 | 20 | 3,59 | 0,17 | +64 | p<0,01 |
| 45 min | 5 | 20 | 3,68 | 0,19 | +69 | p<0,01 |
| 60 min | 7 | 28 | 3,71 | 0,18 | +76 | p<0,01 |
| 90 min | 4 | 16 | 3,33 | 0,23 | +52 | p<0,01 |
| 120 min | 4 | 16 | 2,91 | 0,21 | +33 | p<0,01 |
| 180 min | 8 | 32 | 2,63 | 0,21 | +20 | p<0,05 |
| 300 min | 8 | 32 | 2,25 | 0,17 | + 3 | — |

TABELLE 1c

Antithrombotische Wirkung von Xanthinderivaten (jeweils 10 mg/kg p.o., n=3) (Spalte a) oder erfindungsgemäße konsekutive Anwendung (10 mg/kg Xanthinderivat, 1 Std. später 1 mg/kg Acetylsalicylsäure p.o., n=3) (Spalte b) im Lasermodell an der Ratte. Aufgetragen ist die prozentuale Verbesserung in Vergleich zu den Placebo-Kontrollen.

Experimentelle Einzelheiten im Text.

| Substanz | a | b |
|---|---|---|
| Kontrolle (nur Acetylsalicylsäure) | — | −10% |
| 1-(5-Oxohexyl)-3-methyl-7-propylxanthin | 42% | 56% |
| 1-(5-Hydroxyhexyl)-3-methyl-7-propylxanthin | 40% | 53% |
| 1-(5-Hydroxyhexyl)-3,7-dimethylxanthin | 52% | 68% |
| 1-Propyl-3-methyl-7-(5-hydroxyhexyl-)-xanthin | 35% | 42% |
| 1-Hexyl-3,7-dimethylxanthin | 39% | 46% |
| 1-Äthyl-3-äthyl-7-(5-oxohexyl-)-xanthin | 41% | 48% |
| 1-n-Butyl-3-n-butyl-7-(2-oxopropyl-)-xanthin | 33% | 50% |
| 1-(5-Hydroxyhexyl)-3-methyl-7-(2-methylpropyl)-xanthin | 23% | 34% |
| 1-(2-Methylpropyl)-3-methyl-7-(5-oxohexyl-)-xanthin | 28% | 39% |
| 1-(5-Oxohexyl)-3-methyl-7-(2-methylpropyl)-xanthin | 28% | 39% |
| 1-(2-Methylpropyl)-3-methyl-7-(5-hydroxyhexyl)-xanthin | 29% | 39% |
| 1-(Isopropyl)-3-methyl-7-(5-hydroxyhexyl-)-xanthin | 31% | 36% |
| 1-(3-Methylbutyl-3-methyl-7-(5-oxohexyl)-xanthin | 26% | 35% |
| 1-(3-Methylbutyl)-3-methyl-7-(5-hydroxyhexyl-)-xanthin | 28% | 37% |
| 1-(3-Oxobutyl)-3-methyl-7-propylxanthin | 40% | 49% |

TABELLE 2

Zeitverlauf der Wirkung von Pentoxifyllin und/oder DL-Lysinmonoacetylsalicylat auf die Thrombozytenaggregation

| Substanz | | Zeitverlauf (min) | Dosis i.v. mg/kg | Aggregations-amplitude E ex vivo |
|---|---|---|---|---|
| I. | ohne Kontrolle | 0 | — | 27,5 |
| | DL-Lysinmono-acetylsalicylat | 14 | 1,5 | |
| | | 30 | | 23,5 |
| | DL-Lysinmono-acetylsalicylat | 35 | 0,5 | |
| | | 45 | | 19,5 |
| | Pentoxifyllin | 90 | 20 | |
| | | 150 | | 20 |
| II. | ohne Kontrolle | 0 | — | 26 |
| | Pentoxifyllin | 15 | 20 | |
| | DL-Lysinmono-acetylsalicylat | 45 | 1,5 | |
| | | 110 | | 0,5 |
| III. | ohne Kontrolle | 0 | — | 23 |
| | Pentoxifyllin | 15 | 10 | |
| | | 45 | | 22 |
| | Pentoxifyllin | 60 | 10 | |
| | | 120 | | 20 |

Die folgende Tabelle 3 zeigt eine Analyse der Freisetzung antiaggregatorischer Aktivität aus der Rattenaorta nach oraler Gabe von Pentoxifyllin und verschiedener Mengen Acetylsalicylsäure. Es zeigte sich insbesondere, daß die Reihenfolge der Medikation keinen Einfluß auf die Hemmung der Freisetzung der antiaggregatorischen Aktivität hat.

TABELLE 3

| Experiment | 1 | 2 | 3 | 4 | 5 | 6 |
|---|---|---|---|---|---|---|
| Aggregations-amplitude E ($x 10^2$) | 0,15 | 0,6 | 0,15 | 0,6 | 0,15 | 0,45 |
| Differenz zum Kontroll-experiment | −0,3 | +0,15 | −0,3 | +0,15 | −0,3 | — |

TABELLE 4

Ulcerogene Wirkung von Arzneimitteln

| Substanz | Acetylsalicyl-säure mg/kg p.o. | Anzahl Tiere n | Anzahl Tiere mit Ulcera n |
|---|---|---|---|
| Acetyl- | 1,25 | 10 | 1 |
| salicyl- | 12,50 | 10 | 2 |
| säure | 25 | 10 | 5 |
| | 50 | 10 | 7 |
| | 100 | 10 | 9 |
| konsekutive Gabe, d.h. jeweils | 1,25 | 10 | 1 |
| 100 mg/kg Pentoxi-fyllin $1^h$ | 12,5 | 10 | 2 |
| vor Acetyl-salicyl-säure | 25 | 10 | 5 |
| | 50 | 10 | 7 |
| | 100 | 10 | 8 |

TABELLE 5

Einfluß verschiedener Medikamente auf Blutparameter der arthritischen Ratte (n = 8 pro Gruppe) (Einzelheiten siehe Text)

| Arthritisratte | Erythrozyten-filtrier-barkeit | Thrombose-neigung (Anzahl der Schüsse) | induz. Thrombozyten-aggregation |
|---|---|---|---|
| unbehandelt | 59% | 46% | 100% |
| 10 mg Acetylsalicylsäure | 63% | 47% | 62% |
| 180 mg Acetylsalicylsäure | 68% | 91% | 35% |
| 30 mg Pentoxifyllin | 79% | 87% | 94% |
| 30 mg Pentoxifyllingleichzeitig 10 mg Acetylsalicylsäure | 84% | 57% | 93% |
| 30 mg Pentoxifyllingleichzeitig 180 mg Acetylsalicylsäure | 86% | 91% | 43% |
| 30 mg Pentoxifyllin, nach 1 Stunde 10 mg Acetylsalicylsäure | 90% | 109% | 22% |
| 30 mg Pentoxifyllin, nach 1 Stunde 180 mg Acetylsalicylsäure | 96% | 104% | 30,5% |
| (gesunde Kontrollratten) | 100% | 100% | 0%) |

TABELLE 6

Prozentuale Verbesserung der mit den verschiedenen Medikamenten behandelten arthritischen Ratten im Vergleich zu unbehandelten arthritischen Ratten (n = 8 pro Gruppe) (Einzelheiten seihe Text)

| | Pfotenoedem-Volumen linke Pfote | Pfotenoedem-Volumen rechte Pfote | Nekrose-Index |
|---|---|---|---|
| 10 mg Acetylsalicylsäure | 2 | 3 | 8 |
| 180 mg Acetylsalicylsäure | 28 | 26 | 28 |
| 30 mg Pentoxifyllin | 3 | 0 | 7 |
| 30 mg Pentoxifyllin/ 10 mg Acetylsalicylsäure | 3 | 0 | 4 |
| 30 mg Pentoxifyllin/ 180 mg Acetylsalicylsäure | 10 | 13 | 28 |
| 30 mg Pentoxifyllin/ nach 1 Stunde 10 mg Acetylsalicylsäure | 9 | 12 | 19 |
| 30 mg Pentoxifyllin/ nach 1 Stunde 180 mg Acetylsalicylsäure | 34 | 32 | 34 |

**Patentansprüche für die Vertragsstaaten: BE CH DE FR GB IT LI LU NL SE**

1. Pharmaceutische Zubereitung, enthaltend
A) einerseits ein Xanthinderivat der Formel

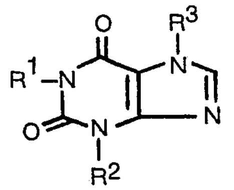

(I)

in der einer der Reste $R^1$ und $R^3$ eine geradkettige Alkyl-, (ω-1)-Oxoalkyl- oder (ω-1)-Hydroxyalkylgruppe mit 3 bis 8 C-Atomen und die beiden anderen Reste $R^2$ und $R^3$ oder $R^1$ und $R^2$ geradkettige oder verzweigte Alkylgruppen mit 1 bis 8 C-Atomen in der Position von $R^1$ und $R^3$ und 1 bis 4 C-Atomen in der Position von $R^2$ darstellen, wobei die Summe der C-Atome dieser beiden Alkylsubstituenten höchstens 10 beträgt, bzw. der Formel

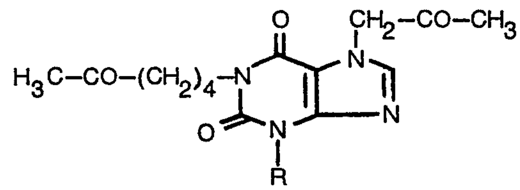

(II)

in der R für einen Alkylrest mit 1 bis 4 C-Atomen steht, bzw. von Prodrug-Formen der Oxoalkylxanthine der Formeln I und II bzw. der Hydroxyalkylxanthine der Formel I, bzw. einen Metabolit eines Xanthinderivats der Formel I bzw. IIO und

B) andererseits O-Acetylsalicylsäure bzw. deren pharmakologisch verträgliche Salze

C) mit oder ohne einen pharmazeutischen Träger zur Anwendung in der Therapie von Krankheiten, die durch gestörte Blutbestandteile, insbesondere Thrombocyten bzw. Erythrocyten, aber auch Leukocyten verursacht bzw. gekennzeichnet sind, derart formuliert, daß die Komponente A) zuerst von der Zubereitung freigesetzt wird, und die Komponente B) 10 Minuten bis 4 Stunden danach von der Zubereitung freigesetzt wird.

2. Verfahren zur Herstellung von pharmazeutischen Zubereitungen, dadurch gekennzeichnet, daß man A) ein Xanthinderivat, das die Formel I bzw. II gemäß Anspruch 1 hat, bzw. eine Prodrug-Form eines Oxoalkylxanthins der Formel I bzw. II bzw. eines Hydroxyalkylxanthins der Formel I bzw. einen Metaboliten eines Xanthinderivats der Formel I bzw. II und B) O-Acetylsalicylsäure bzw. deren pharmakologisch verträglichen Salzen, C) mit oder ohne pharmazeutische Träger in üblicher Weise zu Mental- oder Schichttabletten oder Zäpfchen mit dem Xanthinderivat A) in der äußeren Schicht und der Komponente B) im Kern bzw. der anderen Schicht verarbeitet oder als Kapsel formuliert, derart, daß die Komponente A) zuerst, und die Komponente B) 10 Minuten bis 4 Stunden danach von Mantel- oder Schichttablette, Kapsel oder von dem Zäpfchen freigesetzt wird.

3. Ausführungsform nach Anspruch 1 oder 2, dadurch gekennzeichnet, daß solche Xanthinverbindungen der Formel I zugegen sind, in denen $R^1$ oder $R^3$ einen Alkyl-, (ω-1)-Oxoalkyl- oder (ω-1)-Hydroxyalkylrest mit 5 oder 6 C-Atomen bedeutet und die beiden Alkylsubstituenten $R^2$ und $R^3$ bzw. $R^1$ und $R^2$ zusammen 2 bis 6 C-Atome umfassen.

4. Ausführungsform nach Anspruch 3, dadurch gekennzeichnet, daß das Xanthinderivat in der Position von $R^1$ von $R^3$ eine Hexyl-, 5-Oxohexyl- oder 5-Hydroxyhexylgruppe trägt und vorzugsweise 1-Hexyl-3,7-dimethyl-xanthin, 1-(5-Hydroxyhexyl)-3,7-dimethylxanthin, 1-(5-Oxohexyl)-3,7-dimethyl-xanthin, 1,3-Dimethyl-7-(5-hydroxyhexyl)-xanthin, 1,3-Dimethyl-7-(5-oxohexyl)-xanthin, 1-(5-Hydroxyhexyl)-3-methyl-7-propylxanthin oder 1-(5-Oxohexyl)-3-methyl-7-propylxanthin ist.

5. Ausführungsform nach einem oder mehreren der Ansprüche 1 bis 4, dadurch gekennzeichnet, daß das Xanthinderivat in Prodrug-Form als acetalisiertes Oxoalkylxanthin vorhanden ist, in dem wenigstens eine Carbonylgruppe durch das Strukturelement der Formel

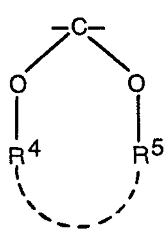

(III)

ersetzt ist oder als O-acyliertes Hydroxyalkylxanthin mit dem Strukturelement der Formel $R^6$—CO—O— (IV), wobei $R^4$ und $R^5$ jeweils eine Alkylgruppe mit bis zu 4 C-Atomen oder zusammen eine Äthylen-, Trimethylen- oder Tetramethylengruppe darstellen und $R^6$ einen Alkylrest mit bis zu 4 C-Atomen, Phenyl, substituiertes Phenyl, Pyridyl oder substituiertes Pyridyl bedeutet.

6. Ausführungsform nach einem oder mehreren der Ansprüche 1 bis 5, dadurch gekennzeichnet, daß das Präparat in einer oral, peroral oder rektal zu verabreichenden Dosiseinheit vorliegt, vorzugsweise einer solchen, die 100 bis 600, vorzugsweise 200 bis 400 mg Pentoxifyllin und 10 bis 2000 mg Acetylsalicylsäure bzw. eine entsprechende Menge eines Salzes davon enthält.

7. Ausführungsform nach einem oder mehreren der Ansprüche 1 bis 6, dadurch gekennzeichnet, daß der Gewichtsanteil des Xanthinderivates, bezogen auf ein Gewichtsteil Acetylsalicylsäure, zwischen etwa 0,1 und 50, vorzugsweise zwischen etwa 0,5 und etwa 50, und insbesondere zwischen etwa 2 und etwa 10 liegt.

8. Ausführungsform nach einem oder mehreren der Ansprüche 1 bis 7, dadurch gekennzeichnet, daß das Trägermaterial für die Acetylsalicylsäure-Komponente Retardierungsmittel enthält.

9. Ausführungsform nach einem oder mehreren der Ansprüche 1 bis 8, dadurch gekennzeichnet, daß das Präparat in Form von Mikrokapseln vorliegt, wobei das Kapselmaterial für die Acetylsalicylsäure-Komponente magensaftresistent ist oder eine vorzögerte Freisetzung ermöglicht.

10. Ausführungsform nach einem oder mehreren der Ansprüche 1 bis 8, dadurch gekennzeichnet, daß das Präparat in Form einer Mantel- oder Schichttablette vorliegt, in der das Xanthinderivat in der schneller zu resorbierenden Schicht enthalten ist.

11. Ausführungsform nach einem oder mehreren der Ansprüche 1 bis 8, dadurch gekennzeichnet, daß das Xanthinderivat A) und die Acetylsalicylsäure-Komponenten B) — letztere in entsprechend verzögerter Form — in voneinander getrennten Dosiseinheiten zur gleichzeitigen Verabreichung vorliegen.

**Patentansprüche für den Vertragsstaat: AT**

1. Verfahren zur Herstellung von pharmazeutischen Zubereitungen, dadurch gekennzeichnet, daß man A) ein Xanthinderivat der Formel

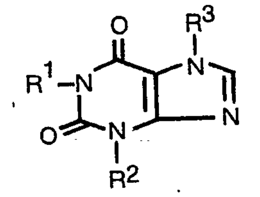

(I)

in der einer der Reste $R^1$ und $R^3$ eine geradkettige Alkyl-, (ω-1)-Oxoalkyl- oder (ω-1)-Hydroxyalkylgruppe mit 3 bis 8 C-Atomen und die beiden anderen Reste $R^2$ und $R^3$ oder $R^1$ und $R^2$ geradkettige oder verzweigte Alkylgruppen mit 1 bis 8 C-Atomen in der Position von $R^1$ und $R^3$ und 1 bis 4 C-Atomen in der Position von $R^2$ darstellen, wobei die Summe der C-Atome dieser beiden Alkylsubstituenten höchstens 10 beträgt, bzw. der Formel

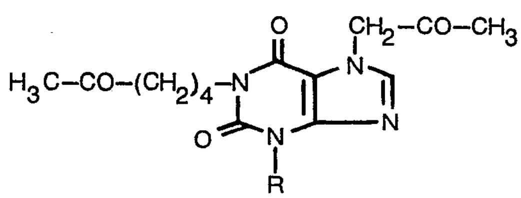

(II)

in der R für einen Alkylrest mit 1 bis 4 C-Atomen steht, bzw. eine Prodrug-Form eines Oxoalkylxanthins der Formel I bzw. II bzw. eines Hydroxyalkylxanthins der Formel I, bzw. einen Metaboliten eines Xanthinderivats der Formel I bzw. IIO und

B) O-Acetylsalicylsäure bzw. deren pharmakologisch verträgliche Salze

C) mit oder ohne pharmazeutische Träger in üblicher Weise zu Mentel- oder Schichttabletten oder Zäpfchen mit dem Xanthinderivat A) in der äußeren Schicht und der Koponente B) im Kern bzw. der anderen Schicht verarbeitet oder als Kepsel(n) formuliert, derart, daß die Komponente A) zuerst und die Komponente B) 10 Minuten bis 4 Stunden danach von der Mantel- oder Schichttablette oder Kepsel oder von dem Zäpfchen freigesetzt wird.

2. Verfahren nach Anspruch 1, dadurch gekennzeichnet, daß solche Xanthinverbindungen der Formel I zugegen sind, in denen $R^1$ oder $R^3$ einen Alkyl-, (ω-1)-Oxoalkyl- oder (ω-1)-Hydroxyalkylrest mit 5 oder 6 C-Atomen bedeutet und die beiden Alkylsubstituenten $R^2$ und $R^3$ bzw. $R^1$ und $R^2$ zusammen 2 bis 6 C-Atome umfassen.

3. Verfahren nach Anspruch 3, dadurch gekennzeichnet, daß das Xanthinderivat in der Position von $R^1$ von $R^3$ eine Hexyl-, 5-Oxohexyl- oder 5-Hydroxyhexylgruppe trägt und vorzugsweise 1-Hexyl-3,7-dimethyl-xanthin, 1-(5-Hydroxyhexyl)-3,7-dimethylxanthin, 1-(5-Oxohexyl)-3,7-dimethyl-xanthin, 1,3-Dimethyl-7-(5-hydroxyhexyl)-xanthin, 1,3-Dimethyl-7-(5-oxohexyl)-xanthin, 1-(5-Hydroxyhexyl)-3-methyl-7-propylxanthin

oder 1-(5-Oxohexyl)-3-methyl-7-propylxanthin ist.

4. Verfahren nach einem oder mehreren der Ansprüche 1 bis 4, dadurch gekennzeichnet, daß das Xanthinderivat in Prodrug-Form als acetalisiertes Oxoalkylxanthin vorhanden ist, in dem wenigstens eine Carbonylgruppe durch das Strukturelement der Formel

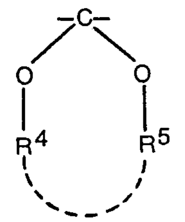

(III)

ersetzt ist oder als O-acyliertes Hydroxyalkylxanthin mit dem Strukturelement der Formel $R^6$—CO—O— (IV), wobei $R^4$ und $R^5$ jeweils eine Alkylgruppe mit bis zu 4 C-Atomen oder zusammen eine Äthylen-, Trimethylen- oder Tetramethylengruppe darstellen und $R^6$ einen Alkylrest mit bis zu 4 C-Atomen, Phenyl, substituiertes Phenyl, Pyridyl oder substituiertes Pyridyl bedeutet.

5. Verfahren nach einem oder mehreren der Ansprüche 1 bis 4, dadurch gekennzeichnet, daß die Zubereitung in einer oral, peroral oder rektal zu verabreichenden Dosiseinheit vorliegt, vorzugsweise einer solchen, die 100 bis 600, vorzugsweise 200 bis 400 mg Pentoxifyllin und 10 bis 2000 mg Acetylsalicylsäure bzw. eine entsprechende Menge eines Salzes davon enthält.

6. Verfahren nach einem oder mehreren der Ansprüche 1 bis 5, dadurch gekennzeichnet, daß der Gewichtsanteil des Xanthinderivates, bezogen auf ein Gewichtsteil Acetylsalicylsäure, zwischen etwa 0,1 und 50, vorzugsweise zwischen etwa 0,5 und etwa 50, und insbesondere zwischen etwa 2 und etwa 10 liegt.

7. Verfahren nach einem oder mehreren der Ansprüche 1 bis 6, dadurch gekennzeichnet, daß das Trägermaterial für die Acetylsalicylsäure-Komponente Retardierungsmittel enthält.

8. Verfahren nach einem oder mehreren der Ansprüche 1 bis 7, dadurch gekennzeichnet, daß das Zubereitung in Form von Mikrokapseln vorliegt, wobei das Kapselmaterial für die Acetylsalicylsäure-Komponente magensaftresistent ist oder eine vorzögerte Freisetzung ermöglicht.

9. Verfahren nach einem oder mehreren der Ansprüche 1 bis 8, dadurch gekennzeichnet, daß die Zubereitung in Form einer Mantel- oder Schichttablette vorliegt, in der das Xanthinderivat in der schneller zu resorbierenden Schicht enthalten ist.

10. Verfahren nach einem oder mehreren der Ansprüche 1 bis 7, dadurch gekennzeichnet, daß das Xanthinderivat A) und die Acetylsalicylsäure-Komponenten B) — letztere in entsprechend verzögerter Form — in voneinander getrennten Dosiseinheiten zur gleichzeitigen Verabreichung vorliegen.

**Revendications pour les Etats contractants: BE CH DE FR GB IT LI LU NL SE**

1. Composition pharmaceutique, contenant

A) d'une part un dérivé xanthinique de formule

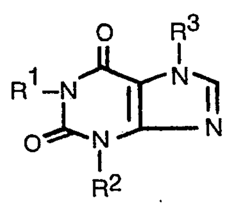

(I)

dans laquelle un des restes $R^1$ et $R^3$ représentent un groupe alkyle, (Ω-1)-oxoalkyle ou (Ω-1)-hydroxyalkyle linéaires à 3 à 8 atomes de carbone et les deux autres restes $R^2$ et $R^3$ ou $R^1$ et $R^2$ représentent des groupes alkyle linéaires ou ramifiés à 1 à 8 atomes de carbone pour $R^1$ et $R^3$ et 1 à 4 atomes de carbone pour $R^2$, la somme des atomes de carbone de ces deux substituants alkyle étant au maximum de 10, ou de formule

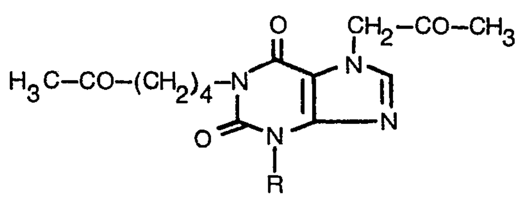

(II)

dans laquelle R est un groupe alkyle à 1 à 4 atomes de carbone, ou bien des formes promédicaments ("prodrugs") des oxoalkylxanthines des formules I et II ou des hydroxyalkylxanthines de formule I, ou encore un métabolite du dérivé xanthinique de formule I ou II, et

B) d'autre part, de l'acide O-acétylsalicylique ou ses sels pharmaceutiquement acceptables

C) avec ou sans support pharmaceutique pour l'administration dans le traitement de maladies, causées ou caractérisées par des perturbations des constituants sanguins, en particulier des thrombocytes ou des érythrocytes, mais également des leucocytes, préparation formulée de telle sorte que: le composant A) soit d'abord libéré à partir de la préparation, et le composant B) ne soit libéré que 10 minutes à 4 heures après.

2. Procédé de fabrication de préparations pharmaceutiques, caractérisé en ce que l'on transforme, de façon usuelle, en des comprimés enrobés ou stratifiés ou en des suppositoires ayant le composant xanthinique A) dans le couche externe et le composant B) dans le noyau ou dans l'autre couche, ou que l'on formule sous forme de capsules: A) un dérivé xanthinique ayant la formule I ou II selon la revendication 1, ou un promédicament d'une oxoalkylxanthine de formule I ou II ou d'une hydroxyalkylxanthine de formule I ou un métabolite d'un dérivé de xanthine de formule I ou II et B) de l'acide O-acétylsalicylique ou ses sels pharmacologiquement acceptables, C) avec ou sans support pharmaceutiquement acceptable, de telle sorte que le composant A) soit d'abord libéré, puis le composant B) 10 minutes à 4 heures après, à partir du comprimé enrobé ou stratifié, de la capsule ou du suppositoire.

3. Mode de réalisation selon la revendication 1 ou 2, caractérisé en ce que sont présents des composés xanthiniques de formule I, dans lesquels $R^1$ ou $R^3$ représente un reste alkyle, (Ω-1)-oxoalkyle ou (Ω-1)-hydroxyalkyle ayant 5 ou 6 atomes de carbone et les deux substituants alkyle $R^2$ et $R^3$ ou $R^1$ et $R^2$ renferment ensemble de 2 à 6 atomes de carbone.

4. Mode de réalisation selon la revendication 3, caractérisé en ce que le dérivé xanthinique est substitué, en position $R^1$ ou $R^3$, par un groupe hexyle, 5-oxohexyle ou 5-hydroxyhexyle, et est, de préférence, la 1-hexyl-3,7-diméthylxanthine, la 1-(5-hydroxyhexyl)-3,7-diméthylxanthine, la 1-(5-oxohexyl)-3,7-diméthylxanthine, la 1,3-diméthyl-7-(5-hydroxyhexyl)xanthine, la 1,3-diméthyl-7-(5-oxohexyl)xanthine, la 1-(5-hydroxyhexyl)-3-méthyl-7-propylxanthine ou la 1-(5-oxohexyl)-3-méthyl-7-propylxanthine.

5. Mode de réalisation selon une ou plusieurs des revendications 1 à 4, caractérisé en ce que le dérivé xanthinique est présent en tant que promédicament sous forme d'oxoalkylxanthine acétalisée, dans laquelle au moins 1 groupe carbonyle est remplacé par l'élément structurel de formule

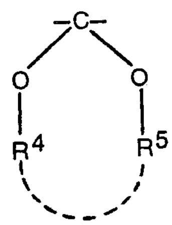

(III)

ou sous forme d'hydroxyalkylxanthine O-acylée avec l'élément structurel de formule $R^6—CO—O—$ (IV), dans laquelle $R^4$ et $R^5$ représentent chaque fois un groupe alkyle ayant jusqu'à 4 atomes de carbone ou forment ensemble un groupe éthylène, triméthylène ou tétraméthylène, et $R^6$ est un groupe alkyle ayant jusqu'à 4 atomes de carbone, phényle substitué ou non, ou pyridyle substitué ou non.

6. Mode de réalisation selon une ou plusieurs des revendications 1 à 5, caractérisé en ce que la préparation se présente sous forme d'une dose unitaire pour administration orale, perorale ou rectale, contenant de préférence 100 à 600, ou mieux 200 à 400 mg de pentoxifylline et 10 à 2000 mg d'acide acétylsalicylique ou une quantité correspondante d'un de leurs sels.

7. Mode de réalisation selon une ou plusieurs des revendications 1 à 6, caractérisé en ce que le pourcentage de dérivé xanthinique, rapporté à la partie en poids d'acide acétylsalicylique, se situe entre environ 0,1 et 50, de préférence entre environ 0,5 et environ 50, et plus particulièrement environ 2 et environ 10.

8. Mode de réalisation selon une ou plusieurs des revendications 1 à 7, caractérisé en ce que le matériau support du composant acide acétylsalicylique contient un agent retard.

9. Mode de réalisation selon une ou plusieurs des revendications 1 à 8, caractérisé en ce que la préparation se présente sous forme de microcapsules, dans lesquelles le matériau est gastrorésistant pour le composant acide acétylsalicylique ou permet une libération retardée.

10. Mode de réalisation selon une ou plusieurs des revendications 1 à 8, caractérisé en ce que la préparation se présente sous forme d'un comprimé enrobé ou stratifié, dans lequel le dérivé xanthinique est contenu dans la couche résorbée plus rapidement.

11. Mode de réalisation selon une ou plusieurs des revendications 1 à 8, caractérisé en ce que le dérivé xanthinique A) et le composant acide acétylsalicylique B), ce dernier sous forme retard appropriée, se présentent en doses unitaires distinctes pour administration simultanée.

**Revendications pour l'Etat contractant: AT**

1. Procédé de fabrication de préparations pharmaceutiques, caractérisé en ce que l'on transforme, de façon usuelle, en des comprimés enrobés ou stratifiés ou en des suppositoires ayant le composant xanthinique A) dans le couche externe et le composant B) dans le noyau ou dans l'autre couche, ou que l'on formule sous forme de capsule(s):

A) un dérivé xanthinique de formule

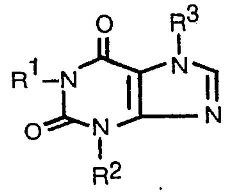

(I)

dans laquelle un des restes $R^1$ et $R^3$ représentent un groupe alkyle, (Ω-1)-oxoalkyle ou (Ω-1)-hydroxyalkyle linéaires à 3 à 8 atomes de carbone et les deux autres restes $R^2$ et $R^3$ ou $R^1$ et $R^2$ représentent des groupes alkyle linéaires ou ramifiés à 1 à 8 atomes de carbone pour $R^1$ et $R^3$ et 1 à 4 atomes de carbone pour $R^2$, la somme des atomes de carbone de ces deux substituants alkyle étant au maximum de 10, ou de formule

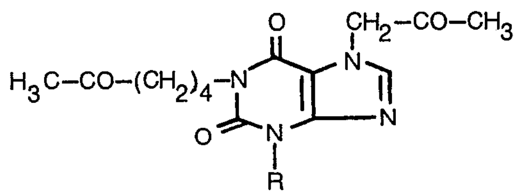

(II)

dans laquelle R est un groupe alkyle à 1 à 4 atomes de carbone, ou bien une forme promédicament ("prodrug") d'une oxoalkylxanthine de formule I ou II ou d'une hydroxyalkylxanthine de formule I, ou encore un métabolite du dérivé xanthinique de formule I ou II, et

B) de l'acide O-acétylsalicylique ou ses sels pharmaceutiquement acceptables

C) avec ou sans support pharmaceutiques, de telle sorte que le composant A) soit d'abord libéré et le composant B) soit libéré, par le comprimé enrobé ou stratifié ou la capsule ou le suppositoire, entre 10 minutes et 4 heures après.

2. Procédé selon la revendication 1, caractérisé en ce que sont présents des composés xanthiniques de formule I, dans lesquels $R^1$ ou $R^3$ représente un reste alkyle, (Ω-1)-oxoalkyle ou (Ω-1)-hydroxyalkyle ayant 5 ou 6 atomes de carbone et les deux substituants alkyle $R^2$ et $R^3$ ou $R^1$ et $R^2$ renferment ensemble de 2 à 6 atomes de carbone.

3. Procédé selon la revendication 3, caractérisé en ce que le dérivé xanthinique est substitué en $R^1$ ou $R^3$ par un groupe hexyle, 5-oxohexyle ou 5-hydroxyhexyle, et est, de préférence, la 1-hexyl-3,7-diméthylxanthine, la 1-(5-hydroxyhexyl)-3,7-diméthylxanthine, la 1-(5-oxohexyl)-3,7-diméthylxanthine, la 1,3-diméthyl-7-(5-hydroxyhexyl)xanthine, la 1,3-diméthyl-7-(5-oxohexyl)xanthine, la 1-(5-hydroxyhexyl)-3-méthyl-7-propylxanthine et la 1-(5-oxohexyl)-3-méthyl-7-propylxanthine.

4. Procédé selon une ou plusieurs des revendications 1 à 4, caractérisé en ce que le dérivé xanthinique est présent en tant que promédicament sous forme d'oxoalkylxanthine acétalisée, dans laquelle au moins 1 groupe carbonyle est remplacé par l'élément structurel de formule

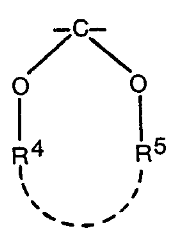

(III)

ou sous forme d'hydroxyalkylxanthine O-acylée avec l'élément structurel de formule $R^6$—CO—O— (IV), dans laquelle $R^4$ et $R^5$ représentent chaque fois un groupe alkyle ayant jusqu'à 4 atomes de carbone ou forment ensemble un groupe éthylène, triméthylène ou tétraméthylène, et $R^6$ est un groupe alkyle ayant jusqu'à 4 atomes de carbone, phényle substitué ou non, ou pyridyle substitué ou non.

5. Procédé selon une ou plusieurs des revendications 1 à 4, caractérisé en ce que la préparation se présente sous forme d'une dose unitaire pour administration orale, perorale ou rectale, contenant de préférence 100 à 600, ou mieux 200 à 400 mg de pentoxifylline et 10 à 2000 mg d'acide acétylsalicylique ou une quantité correspondante d'un de leurs sels.

6. Procédé selon une ou plusieurs des revendications 1 à 5, caractérisé en ce que le pourcentage de dérivé xanthinique, rapporté à la partie en poids d'acide acétylsalicylique, se situe entre environ 0,1 et 50, de préférence entre environ 0,5 et environ 50, et plus particulièrement environ 2 et environ 10.

7. Procédé selon une ou plusieurs des revendications 1 à 6, caractérisé en ce que le matériau support du composant acide acétylsalicylique contient un agent retard.

8. Procédé selon une ou plusieurs des revendications 1 à 7, caractérisé en ce que la préparation se présente sous forme de microcapsules, dans lesquelles le matériau est gastrorésistant pour le composant acide acétylsalicylique ou permet une libération agent retardée.

9. Procédé selon une ou plusieurs des revendications 1 à 8, caractérisé en ce que la préparation se présente sous forme d'un comprimé enrobé ou stratifié, dans lequel le dérivé xanthinique est contenu dans la couche résorbée plus rapidement.

10. Procédé selon une ou plusieurs des revendications 1 à 7, caractérisé en ce que le dérivé xanthinique A) et le composant acide acétylsalicylique B), ce dernier sous forme retard appropriée, se présentent en doses unitaires distinctes pour administration simultanée.

**Claims for the Contracting States: BE CH DE FR GB IT LI LU NL SE**

1. A pharmaceutical formulation containing

A) on the one hand a xanthine derivative of the formula

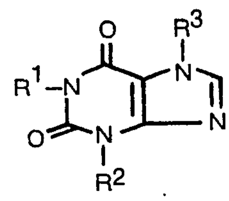

(I)

in which one of the radicals $R^1$ and $R^3$ represents a straight-chain alkyl, (ω-1)-oxoalkyl or (ω-1)-hydroxyalkyl group having 3 to 8 carbon atoms, and the other two radicals $R^2$ and $R^3$, or $R^1$ and $R^2$ represent straight-chain or branched alkyl groups having 1 to 8 carbon atoms in the position of $R^1$ and $R^3$ and 1 to 4 carbon atoms in the position of $R^2$, the maximum total of carbon atoms in these two alkyl substituents being 10, or of the formula

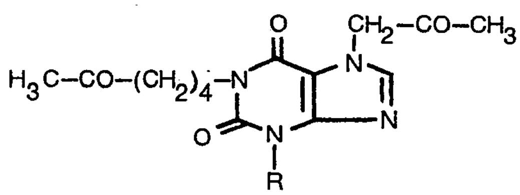

(II)

in which R represents an alkyl radical having 1 to 4 carbon atoms, or prodrug forms of the oxoalkylxanthines of the formulae I and II or of the hydroxyalkylxanthines of the formula I, or a metabolite of a xanthine derivative of the formula I or II, and

B) on the other hand o-acetylsalicylic acid or its pharmacologically tolerated salts,

C) with or without a pharmaceutical vehicle, for use in the treatment of diseases caused or characterized by impaired constituents of blood, in particular platelets or erythrocytes, but also leukocytes, formulated in such a manner that component A) is released first from the formulation, and component B) is released 10 minutes to 4 hours thereafter from the formulation.

2. A process for the preparation of pharmaceutical formulations, which comprises processing A) a xanthine derivative which has the formula I or II as claimed in claim 1, or a prodrug form of an oxoalkylxanthine of the formula I or II, or of a hydroxyalkylxanthine of the formula I, or a metabolite of a xanthine derivative of the formula I or II, and B) o-acetylsalicylic acid or its pharmacologically tolerated salts, C) with or without pharmaceutical vehicles, in a customary manner to give coated or layered tablets or suppositories with the xanthine derivative A) in the outer layer and the component B) in the core or other layer, or formulating them as a capsule in such a manner that component A) is released first and component B) is released 10 minutes to 4 hours thereafter from the coated or layered tablet, capsule or from the suppository.

3. An embodiment as claimed in claim 1 or 2, which contains those xanthine compounds of the formula I in which $R^1$ or $R^3$ denotes an alkyl, (ω-1)-oxoalkyl or (ω-1)-hydroxyalkyl radical having 5 or 6 carbon atoms, and the two alkyl substituents $R^2$ and $R^3$ or $R^1$ and $R^2$ together comprise 2 to 6 carbon atoms.

4. An embodiment as claimed in claim 3, wherein the xanthine derivative carries in the position of $R^1$ or $R^3$ a hexyl, 5-oxohexyl or 5-hydroxyhexyl group, and is preferably 1-hexyl-3,7 dimethylxanthine, 1-(5-hydroxyhexyl)-3,7-dimethylxanthine, 1-(5-oxohexyl)-3,7-dimethylxanthine, 1,3-dimethyl-7-(5-hydroxyhexyl)-xanthine, 1,3-dimethyl-7-(5-oxohexyl)-xanthine, 1-(5-hydroxyhexyl)-3-methyl-7-propylxanthine or 1-(5-oxohexyl)-3-methyl-7-propylxanthine.

5. An embodiment as claimed in one or more of claims 1 to 4, in which the xanthine derivative is present in the prodrug form as acetalized oxoalkylxanthine in which at least one carbonyl group is replaced by the structural element of the formula

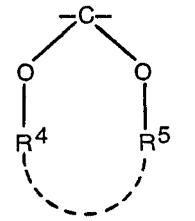

(III)

or as o-acylated hydroxyalkylxanthine having the structural element of the formula $R^6$—CO—o— (IV), $R^4$ and $R^5$ each representing an alkyl group having up to 4 carbon atoms or together representing an ethylene, trimethylene or tetramethylene group, and $R^6$ denoting an alkyl radical having up to 4 carbon atoms, phenyl, substituted phenyl, pyridyl or substituted pyridyl.

6. An embodiment as claimed in one or more of claims 1 to 5, wherein the product is in the form of a dose unit which is to be administered orally, perorally or rectally, preferably one which contains 100 to 600, preferably 200 to 400, mg of pentoxifylline and 10 to 2000 mg of acetylsalicylic acid or an equivalent amount of a salt thereof.

7. An embodiment as claimed in one or more of claims 1 to 6, wherein the proportion by weight of the xanthine derivative relative to one part by weight of acetylsalicylic acid is between about 0.1 and 50, preferably between about 0.5 and about 50, and in particular between about 2 and about 10.

8. An embodiment as claimed in one or more of claims 1 to 7, wherein the vehicle for the acetylsalicylic acid component contains retarding means.

9. An embodiment as claimed in one or more of claims 1 to 8, wherein the product is in the form of microcapsules, the capsule material for the acetylsalicylic acid component being resistant to gastric acid or allowing delayed release.

10. An embodiment as claimed in one or more of claims 1 to 8, wherein the product is in the form of a coated or layered tablet in which the xanthine derivative is contained in the layer which is to be absorbed more rapidly.

11. An embodiment as claimed in one or more of claims 1 to 8, wherein the xanthine derivative A) and the acetylsalicylic acid components B) — the latter are appropriate by delayed form — are present in dose units which are separate from one another but for simultaneous administration.

**Claims for the Contracting State: AT**

1. A process for the preparation of pharmaceutical formulations, which comprises processing
A) a xanthine derivative of the formula

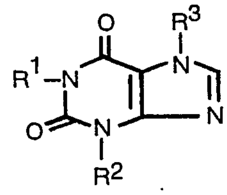

(I)

in which one of the radicals $R^1$ and $R^3$ represents a straight-chain alkyl, (ω-1)-oxoalkyl or (ω-1)-hydroxyalkyl group having 3 to 8 carbon atoms, and the two other radicals $R^2$ and $R^3$, or $R^1$ and $R^2$ represent straight-chain or branched alkyl groups having 1 to 8 carbon atoms in the position of $R^1$ and $R^3$ and 1 to 4 carbon atoms in the position of $R^2$, the maximum total of carbon atoms in these two alkyl substituents being 10, or of the formula

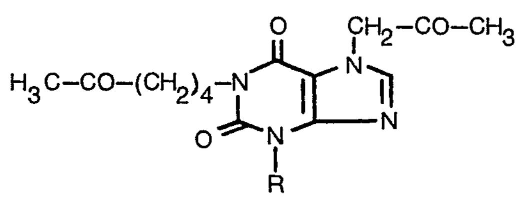

(II)

in which R represents an alkyl radical having 1 to 4 carbon atoms, or a prodrug form of an oxoalkylxanthine of the formula I or II or of a hydroxyalkylxanthine of the formula I, or metabolites of a xanthine derivative of the formula I or II, and

B) o-acetylsalicylic acid or its pharmacologically tolerated salts,

C) with or without a pharmaceutical vehicle, in a customary manner to give coated or layered tablets or suppositories with the xanthine derivative A) in the outer layer and the component B) in the core or other layer, or formulating them as capsule(s) in such a manner that component A) is released first and component B) is released 10 minutes to 4 hours thereafter from the coated or layered tablet or capsule or from the suppository.

2. The process as claimed in claim 1, which contains those xanthine compounds of the formula I in which $R^1$ or $R^3$ denotes an alkyl, (ω-1)-oxoalkyl or (ω-1)-hydroxyalkyl radical having 5 or 6 carbon atoms, and the two alkyl substituents $R^2$ and $R^3$ or $R^1$ and $R^2$ together comprise 2 to 6 carbon atoms.

3. The process as claimed in claim 1, wherein the xanthine derivative carries in the position of $R^1$ or $R^3$ a hexyl, 5-oxohexyl or 5-hydroxyhexyl group, and is preferably 1-hexyl-3,7-dimethylxanthine, 1-(5-hydroxyhexyl)-3,7-dimethylxanthine, 1-(5-oxohexyl)-3,7-dimethylxanthine, 1,3-dimethyl-7-(5-hydroxyhexyl)-xanthine, 1,3-dimethyl-7-(5-oxohexyl)-xanthine, 1-(5-hydroxyhexyl)-3-methyl-7-propylxanthine or 1-(5-oxohexyl)-3-methyl-7-propylxanthine.

4. The process as claimed in one or more of claims 1 to 3, in which the xanthine derivative is present in the prodrug form as acetalized oxoalkylxanthine in which at least one carbonyl group is replaced by the structural element of the formula

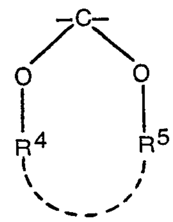

(III)

or as o-acylated hydroxyalkylxanthine having the structural element of the formula $R^6$—CO—o— (IV), $R^4$ and $R^5$ each representing an alkyl group having up to 4 carbon atoms or together representing an ethylene, trimethylene or tetramethylene group, and $R^6$ denoting an alkyl radical having up to 4 carbon atoms, phenyl, substituted phenyl, pyridyl or substituted pyridyl.

5. The process as claimed in one or more of claims 1 to 4, wherein the formulation is in the form of a dose unit which is to be administered orally, perorally or rectally, preferably one which contains 100 to 600, preferably 200 to 400, mg of pentoxifylline and 10 to 2000 mg of acetylsalicylic acid or an equivalent amount of a salt thereof.

6. The process as claimed in one or more of claims 1 to 5, wherein the proportion by weight of the xanthine derivative relative to one part by weight of acetylsalicylic acid is between about 0.1 and 50, preferably between about 0.5 and about 50, and in particular between about 2 and about 10.

7. The process as claimed in one or more of claims 1 to 6, wherein the vehicle for the acetylsalicylic acid component contains retarding means.

8. The process as claimed in one or more of claims 1 to 7, wherein the formulation is in the form of microcapsules, the capsule material for the acetylsalicylic acid component being resistant to gastric acid or allowing delayed release.

9. The process as claimed in one or more of claims 1 to 8, wherein the formulation is in the form of a coated or layered tablet in which xanthine derivative is contained in the layer which is to be absorbed more rapidly.

10. The process as claimed in one or more of claims 1 to 7, wherein the xanthine derivative A) and the acetylsalicylic acid components B) — the latter are appropriate by delayed form — are present in dose units which are separate from one another but for simultaneous administration.